(19) Europäisches Patentamt
European Patent Office
Office européen des brevets

(11) **EP 4 119 161 A1**

(12) **EUROPEAN PATENT APPLICATION**
published in accordance with Art. 153(4) EPC

(43) Date of publication:
**18.01.2023 Bulletin 2023/03**

(21) Application number: **21768737.5**

(22) Date of filing: **10.03.2021**

(51) International Patent Classification (IPC):
*A61K 39/395* $^{(2006.01)}$    *A61K 9/08* $^{(2006.01)}$
*A61K 47/22* $^{(2006.01)}$    *A61K 47/26* $^{(2006.01)}$
*A61K 47/12* $^{(2006.01)}$    *C07K 16/24* $^{(2006.01)}$

(52) Cooperative Patent Classification (CPC):
**A61K 9/08; A61K 39/395; A61K 47/12;**
**A61K 47/22; A61K 47/26; C07K 16/24**

(86) International application number:
**PCT/KR2021/002983**

(87) International publication number:
**WO 2021/182874 (16.09.2021 Gazette 2021/37)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **13.03.2020 KR 20200031611**

(71) Applicant: **Samsung Bioepis Co., Ltd.**
**Yeonsu-gu**
**Incheon 21987 (KR)**

(72) Inventors:
• **KIM, Mi Gyeong**
**Anyang-si, Gyeonggi-do 14061 (KR)**
• **LEE, Seung Ha**
**Incheon 22008 (KR)**
• **KIM, Han Soo**
**Incheon 21998 (KR)**
• **KIM, In Ae**
**Seoul 05090 (KR)**
• **LEE, Na Young**
**Incheon 21986 (KR)**
• **KIM, Soo Shin**
**Seoul 08275 (KR)**
• **KIM, Ji Hyun**
**Seoul 04129 (KR)**

(74) Representative: **Boult Wade Tennant LLP**
**Salisbury Square House**
**8 Salisbury Square**
**London EC4Y 8AP (GB)**

(54) **LIQUID PHARMACEUTICAL COMPOSITION HAVING IMPROVED STABILITY**

(57)    Provided is a liquid pharmaceutical composition having increased stability, and more particularly, a stable liquid pharmaceutical composition including a protein.

FIG. 1

EP 4 119 161 A1

**Description**

TECHNICAL FIELD

The present disclosure relates to a liquid pharmaceutical composition having increased stability, and more particularly, a stable liquid pharmaceutical composition including a protein.

BACKGROUND ART

The treatment of tumor necrosis factor-alpha (TNF-α)-related autoimmune diseases, e.g., rheumatoid arthritis, psoriasis, and other autoimmune diseases, has been achieved by the use of FDA-approved drugs such as adalimumab (HUMIRA®, Abbvie Corporation). Adalimumab is a human monoclonal antibody that down-regulates inflammatory responses associated with autoimmune diseases by inhibiting human TNF-α activity and preventing it from activating TNF receptors. The approved medical indications for Adalimumab include rheumatoid arthritis, psoriatic arthritis, ankylosing spondylitis, Crohn's disease, ulcerative colitis, moderate to severe chronic psoriasis, and juvenile idiopathic arthritis.

Adalimumab is a recombinant human immunoglobulin G1 monoclonal antibody that selectively binds to TNF-α in the body, thereby inhibiting the immune response by tumor necrosis factor-alpha. This antibody is also known as D2E7. Adalimumab consists of 1330 amino acids with a molecular weight of about 148 kilodaltons. Adalimumab is disclosed and claimed in US Patent No. 6,090,382, the disclosure of which is incorporated herein by reference in its entirety.

It is very important to prepare protein drugs, such as antibodies, into suitable formulations such that they do not lose physiological activity during a period of storage and/or preservation, the proteins are not fragmented and/or agglomerated or granulated, and their denaturation, such as by oxidation/reduction, does not occur. Therefore, studies on various formulations of protein drugs are actively being conducted.

An object of the present disclosure is to develop a stable formulation capable of reducing instability of an antibody protein when developing adalimumab biosimilars.

DESCRIPTION OF EMBODIMENTS

TECHNICAL PROBLEM

Accordingly, the present disclosure provides an aqueous liquid composition for increasing stability of a protein drug, specifically, a pharmaceutical composition including a protein drug.

An aspect provides a pharmaceutical composition including

an anti-TNFa antibody;
histidine
; and
polysorbate 20
,
wherein pH is about 4.0 to about 8.0 or about 5.0 to about 5.5.

The pharmaceutical composition may have one or more of the following characteristics:

(i) improvement in photostability, as compared with a histidine-free composition; and
(ii) improvement in thermal stability, freeze-thaw stability, or both thermal stability and freeze-thaw stability, as compared with a composition containing polysorbate 80 instead of polysorbate 20.

The histidine-free means that histidine is not included in a detectable amount or in an amount sufficient to exhibit a significant effect, and histidine is not completely included (about 0 mM), or is not substantially included (e.g., about 50 nM or less, about 40 nM or less, about 30 nM or less, about 20 nM or less, about 10 nM or less, about 5 nM or less, about 1 nM or less).

Another aspect provides a container including the pharmaceutical composition, wherein the pharmaceutical composition has stability against temperature changes.

The pharmaceutical composition provided in the present disclosure may further include a buffer solution. The buffer solution may have pH of about 4.0 to about 8.0 or pH of about 5.0 to about 5.5. The buffer solution may be one or more, or two or more (e.g., one, two, three, or four) selected from the group consisting of citrate, phosphate, acetate, and succinate. In an embodiment, the buffer solution may include citrate. In another embodiment, the buffer solution may include one or more selected from the group consisting of phosphate, succinate, and acetate. In this regard, the buffer solution may be free of citrate. In one specific embodiment, the buffer solution may include phosphate. In another specific embodiment, the buffer solution may include phosphate, and may be free of citrate. In another specific embodiment, the buffer solution may include (1) phosphate, and (2) succinate or acetate. In another embodiment, the buffer solution may include (1) phosphate, and (2) succinate or acetate, and may be free of citrate.

The pharmaceutical composition provided in the present disclosure may further include a polyol. In the present disclosure, the polyol may be a tonicity agent or a stabilizer. The polyol may be one or more selected from the group consisting of sugar and sugar alcohols. Specifically, the polyol may be one or more selected from the group consisting of sorbitol, mannitol, meglumine, trehalose, sucrose, maltose, lactose, glucose, xylitol, arabitol, erythritol, lactitol, maltitol, inositol, etc. In an embodiment, the polyol may be sorbitol, mannitol, or a combination thereof. In an embodiment, the polyol may include sorbitol, and may be free of one or more selected from sugars and sugar alcohols (e.g., mannitol, meglumine, trehalose, sucrose, maltose, lactose, glucose, xylitol, arabitol, erythritol, lactitol, maltitol, inositol, etc.) other than sorbitol, or all of them, and for example, may be free of mannitol. In another embodiment, the polyol may include mannitol, and may be free of one or more selected from sugars and sugar alcohols (e.g., sorbitol, meglumine, trehalose, sucrose, maltose, lactose, glucose, xylitol, arabitol, erythritol, lactitol, maltitol, inositol, etc.) other than mannitol, or all of them, and for example, may be free of sorbitol.

The pharmaceutical composition provided in the present disclosure may be free of polysorbates (e.g., polysorbate 80) other than polysorbate 20.

The pharmaceutical composition may further include a buffer solution and a polyol. The buffer solution and the polyol are the same as described above.

In an embodiment, the pharmaceutical composition may further include citrate and sorbitol. In another embodiment, the pharmaceutical composition may further include citrate and sorbitol, and may be free of one or more selected from sugars and sugar alcohols other than sorbitol, or all of them.

In another embodiment, the pharmaceutical composition may further include phosphate and mannitol. In another embodiment, the pharmaceutical composition may further include phosphate and mannitol, and may be free of citrate. In another embodiment, the pharmaceutical composition may further include phosphate and mannitol, and may be free of one or more selected from sugars and sugar alcohols other than mannitol, or all of them. In another embodiment, the pharmaceutical composition may further include phosphate and mannitol, may be free of citrate, and may be free of one or more selected from sugars and sugar alcohols other than mannitol, or all of them.

In another embodiment, the pharmaceutical composition may further include (1) phosphate, (2) succinate or acetate, and (3) mannitol. In another embodiment, the pharmaceutical composition may further include (1) phosphate, (2) succinate or acetate, and (3) mannitol, and may be free of citrate. In another embodiment, the pharmaceutical composition may further include (1) phosphate, (2) succinate or acetate, and (3) mannitol, and may be free of one or more selected from sugars and sugar alcohols other than mannitol, or all of them. In another embodiment, the pharmaceutical composition may further include (1) phosphate, (2) succinate or acetate, and (3) mannitol, may be free of citrate, and may be free of one or more selected from sugars and sugar alcohols other than mannitol, or all of them.

Still another aspect provides a device or a container, each including the pharmaceutical composition.

Still another aspect provides a device or a container for treatment of a disease, each including the pharmaceutical composition. The disease may be a disease on which an anti-TNFa antibody, e.g., adalimumab exhibits therapeutic, ameliorating, and/or prophylactic effects.

Still another aspect provides a method of treating a disease, the method including administering the pharmaceutical composition to a subject in need of administration of an anti-TNFa antibody, e.g., adalimumab. The administering may be performed using the device or container including the pharmaceutical composition.

SOLUTION TO PROBLEM

The present disclosure provides an aqueous liquid composition for increasing stability of a protein drug, specifically, a liquid pharmaceutical composition including a protein drug. The aqueous liquid composition (liquid pharmaceutical composition) may include components described below, and a residual amount of aqueous medium (water (purified water), physiological saline, sterile water for injection, etc.).

Definitions

As used herein, the term "about" or "approximately" may be generally interpreted as including a value or range within $\pm 20\%$, $\pm 10\%$, $\pm 5\%$, $\pm 4\%$, $\pm 3\%$, $\pm 2\%$, or $\pm 1\%$ of a given value or range.

The term "long-term storage stability" or "long-term stability" may mean that a pharmaceutical composition may be stored in a stable state for three months or more, six months or more, one year or more, or two years or more. For example, the term "storage" may be understood as a concept that embraces storage of a pharmaceutical composition under stress conditions, such as storage at 2 °C to 8 °C in a liquid-phase, frozen at -20 °C or lower, or subjected to one or more freeze-thaw cycles.

The term "stable state" may be understood as a state wherein a protein (e.g., adalimumab) included in a pharmaceutical composition exhibits loss in its biological activity and/or structural stability (e.g., aggregation, degradation, denaturation (acidic or basic), oxidation, etc.) during the period of storage by 20% or less, 15% or less, 10% or less, or 5% or less, as compared with that of the initial storage.

The term "free of A" or "substantially free of A" may be understood to mean that A is completely absent or exists in a small amount without any substantial effect on properties of the composition. When the amount of A is not mentioned, it may be understood to mean an "undetectable amount". Unless otherwise specified in the specification, "free of A" may be interpreted as including "substantially free of A" including the case where A is completely absent.

(1) Anti-TNFa antibody

In the present disclosure, the anti-TNFa antibody refers to an antibody that binds to TNFα and regulates biological activities thereof. Tumor necrosis factor-alpha (TNF-alpha, TNFα) is a cytokine produced by various kinds of cells, such as monocytes and macrophages, by stimulation with endotoxin, etc. TNFα, which activates TNF receptors to induce reactions such as T-cell activation, thymocyte proliferation, etc., is a key mediator of major inflammatory, immunological, and pathophysiological responses.

The anti-TNFa antibody may be in the form of a full-length antibody or an antibody fragment including an antigen-binding site thereof, but is not particularly limited thereto. Specifically, the anti-TNFa antibody may be a human immunoglobulin G1 monoclonal antibody, and more specifically, may be adalimumab.

Adalimumab is the first intact human antibody developed as a drug and was derived from a phage display technique, with the enhanced affinity thereof resulting from a modification in CDR. Adalimumab, also called D2E7, consists of 1330 amino acids with a molecular weight of about 148 kD. Adalimumab has been commercially available under the brand name of HUMIRA. HUMIRA has been approved for sale as a therapeutic agent for rheumatoid arthritis and applied for the treatment of Crohn's disease, ankylosing spondylitis, psoriatic arthritis, ulcerative colitis, etc. For more detailed information on adalimumab, a person skilled in the art could easily obtain the information from well-known database.

As used herein, the term "adalimumab" may also be interpreted as including adalimumab that is modified in the amino acid structure (deletion, addition, and/or substitution of amino acids) and/or in glycosylation property within a range that does not affect polypeptide functions.

In the pharmaceutical composition provided in the present disclosure, the anti-TNFα antibody may be included in a therapeutically effective amount. Specifically, the therapeutically effective amount may be about 25 mg/ml to about 200 mg/ml, about 25 mg/ml to about 175 mg /ml, about 25 mg/ml to about 150 mg/ml, about 25 mg/ml to about 125 mg/ml, about 25 mg/ml to about 100 mg/ml, about 25 mg/ml to about 75 mg/ml, about 25 mg/ml to about 50 mg/ml, about 30 mg/ml to about 200 mg/ml, about 30 mg/ml to about 175 mg /ml, about 30 mg/ml to about 150 mg/ml, about 30 mg/ml to about 125 mg/ml, about 30 mg/ml to about 100 mg/ml, about 30 mg/ml to about 75 mg/ml, about 30 mg/ml to about 50 mg/ml, about 50 mg/ml to about 200 mg/ml, about 50 mg/ml to about 175 mg /ml, about 50 mg/ml to about 150 mg/ml, about 50 mg/ml to about 125 mg/ml, about 50 mg/ml to about 100 mg/ml, about 75 mg/ml to about 200 mg/ml, about 75 mg/ml to about 175 mg/ml, about 75 mg/ml to about 150 mg/ml, about 75 mg/ml to about 125 mg/ml, about 75 mg/ml to about 100 mg/ml, about 100 mg/ml to about 200 mg/ml, about 100 mg/ml to about 175 mg/ml, about 100 mg/ml to 150 mg/ml, about 100 mg/ml to about 125 mg/ml, about 150 mg/ml to about 200 mg/ml, about 150 mg/ml to about 175 mg/ml, about 50 mg/ml, about 100 mg/ml, or about 200 mg/ml.

(2) Amino acid

Histidine included in the pharmaceutical composition provided in the present disclosure may function as a stabilizer or a buffer solution, and may be included at a concentration of about 50 mM or more, about 51 mM or more, about 52 mM or more, or about 59 mM or more, e.g., about 50 mM to about 100 mM, about 50 mM to about 89 mM, about 50 mM to about 80 mM, about 50 mM to about 69 mM, about 50 mM to about 65 mM, about 50 mM to about 62 mM, about 50 mM to about 59 mM, about 51 mM to about 100 mM, about 51 mM to about 89 mM, about 51 mM to about 80 mM, about 51 mM to about 69 mM, about 51 mM to about 65 mM, about 51 mM to about 62 mM, about 51 mM to about 59 mM, about 52 mM to about 100 mM, about 52 mM to about 89 mM, about 52 mM to about 80 mM, about 52 mM to about 69 mM, about 52 mM to about 65 mM, about 52 mM to about 62 mM, about 52 mM to about 59 mM, about 54 mM to about 100 mM, about 54 mM to about 89 mM, about 54 mM to about 80 mM, about 54 mM to about 69 mM, about 54 mM to about 64 mM, about 54 mM to about 62 mM, about 54 mM to about 59 mM, about 56 mM to about 100 mM, about 56 mM to about 89 mM, about 56 mM to about 80 mM, about 56 mM to about 69 mM, about 56 mM to about 65 mM, about 56 mM to about 62 mM, about 56 mM to about 59 mM, about 59 mM to about 100 mM, about 59 mM to about 89 mM, about 59 mM to about 80 mM, about 59 mM to about 69 mM, about 59 mM to about 65 mM, about 59 mM to about 62 mM, or about 59 mM, based on the total pharmaceutical composition.

The histidine may be included in the form of histidine and/or in the form of a pharmaceutically acceptable salt (e.g., hydrochloride, etc.) thereof and/or a hydrate (e.g., monohydrate, etc.) thereof (e.g., histidine hydrochloride monohydrate, etc.), but is not limited thereto. Unless specified otherwise, the term "histidine" may be interpreted as including one or more selected from the group consisting of histidine, a pharmaceutically acceptable salt (e.g., hydrochloride, etc.) thereof, and a hydrate (e.g., monohydrate, etc.) thereof (e.g., histidine hydrochloride monohydrate, etc.).

In the pharmaceutical composition provided in the present disclosure, the histidine may function as an antioxidant agent, an anti-aggregation agent, or both of an anti-oxidant agent and an anti-aggregation agent.

The pharmaceutical composition provided in the present disclosure may be free of or may be substantially free of one or more (e.g., all) selected from the group consisting of amino acids other than histidine, and pharmaceutically acceptable salts thereof. In an embodiment, the pharmaceutical composition provided in the present disclosure may be free of or may be substantially free of one or more (e.g., all of them) selected from the group consisting of arginine, lysine, proline, glycine, methionine, glutamine, and pharmaceutically acceptable salts thereof (e.g., hydrochloride, hydrate, etc.).

(3) Surfactant

In the present disclosure, the surfactant may be selected from all pharmaceutically acceptable surfactants capable of evenly dispersing a protein in a liquid composition medium. The surfactant may be a non-ionic surfactant, and specifically, one or more selected from the group consisting of polysorbates (e.g., polysorbate 20 (polyoxyethylene (20) sorbitan monolaurate), polysorbate 40 (polyethylene (20) sorbitan monopalmitate), polysorbate 60 (polyethylene (20) sorbitan monostearate), polysorbate 65 (polyethylene (20) sorbitan tristearate), polysorbate 80 (polyethylene (20) sorbitan monooleate), polysorbate 85 (polyethylene 20 sorbitan trioleate) (the number (20) following polyoxyethylene refers to the total number of oxyethylene units ($-(CH_2CH_2O)-$)), poloxamer (PEO-PPO-PEO copolymer; PEO: poly(ethylene oxide), PPO: poly(propylene oxide)), sorbitan esters (e.g., sorbitan polyethoxylates, etc.), polyethylene-polypropylene glycol, polyoxyethylene compounds (e.g., polyoxyethylene-stearate, polyoxyethylene alkyl ether (alkyl: C1-C30), polyoxyethylene monolauryl ether, alkylphenyl polyoxyethylene copolymer (alkyl: C1-C30), etc.), sodium dodecyl sulphate (SDS), etc. More specifically, to achieve much better formulation stability, the surfactant may be polysorbate 20. In an exemplary embodiment, the formulation of the present disclosure may include polysorbate 20, and may be free of polysorbate 80.

To further improve the protein stability in the formulation of the present disclosure, a concentration of the surfactant, for example, polysorbate 20, included in the pharmaceutical composition may be about 0.01%(w/v) to about 0.2%(w/v), about 0.01%(w/v) to about 0.1%(w/v), about 0.01%(w/v) to about 0.08%(w/v), about 0.03%(w/v) to about 0.2%(w/v), about 0.03%(w/v) to about 0.1%(w/v), about 0.03%(w/v) to about 0.08%(w/v), more than about 0.04%(w/v) and about 0.2%(w/v) or less, more than about 0.04%(w/v) and about 0.1%(w/v) or less, more than about 0.04%(w/v) and about 0.08%(w/v) or less, about 0.05%(w/v) to about 0.2%(w/v), about 0.05%(w/v) to about 0.1%(w/v), about 0.05%(w/v) to about 0.08%(w/v), about 0.01%(w/v), about 0.03%(w/v), about 0.04%(w/v), about 0.05%(w/v), about 0.08%(w/v), about 0.1%(w/v) or about 0.2%(w/v), based on the total pharmaceutical composition.

(4) Buffer solution

In the present disclosure, the buffer solution functions to adjust pH of the formulation, and any buffer solution commonly used in preparing protein formulations may be used without limitation. Specifically, the buffer solution may include one or more, or two or more selected from the group consisting of citrate, phosphate, acetate, succinate, and pharmaceutically acceptable salt thereof, and/or hydrates (e.g., monohydrate, etc.) thereof. The pharmaceutically acceptable salt may be one or more selected from the group consisting of a sodium salt, a potassium salt, a succinate salt, a phosphate salt, etc., but is not limited thereto.

Unless specified otherwise, the term "citrate" may be interpreted as meaning one or more selected from the group consisting of citrate, a pharmaceutically acceptable salt thereof (e.g., sodium citrate), and a hydrate thereof (e.g., citric acid monohydrate), e.g., one or more (e.g., one, two, or three) selected from the group consisting of citrate, sodium citrate, and citric acid monohydrate.

Unless specified otherwise, the term "phosphate" may be interpreted as meaning one or more selected from the group consisting of phosphate, a pharmaceutically acceptable salt (e.g., sodium salt) thereof, and a hydrate thereof.

Unless specified otherwise, the term "succinate" may be interpreted as meaning one or more selected from the group consisting of succinate, a pharmaceutically acceptable salt (e.g., sodium salt) thereof, and a hydrate thereof.

Unless specified otherwise, the term "acetate" may be interpreted as meaning one or more selected from the group consisting of acetate, a pharmaceutically acceptable salt (e.g., sodium salt) thereof, and a hydrate thereof.

To improve stability of the anti-TNF alpha antibody protein included in the pharmaceutical composition provided in the present disclosure, pH of the buffer solution may be about 4.0 to about 8.0, and pH thereof may be about 4.0 to about 7.5, about 4.0 to about 7, about 4.0 to about 6.5, about 4.0 to about 6, about 4.0 to about 5.5, about 4.5 to about 7.5, about 4.5 to about 7, about 4.5 to about 6.5, about 4.5 to about 6, about 4.5 to about 5.5, about 4.8 to about 7.5, about 4.8 to about 7, about 4.8 to about 6.5, about 4.8 to about 6, about 4.8 to about 5.6, about 5.0 to about 7.5, about 5.0 to about 7, about 5.0 to about 6.5, about 5.0 to about 6, about 5.0 to about 5.5, about 5.2 to about 7.5, about 5.2 to about 7.2, about 5.2 to about 7, about 5.2 to about 6.8, about 5.2 to about 6.6, about 5.2 to about 6.5, about 5.5 to about 7.5, about 5.5 to about 7.2, about 5.5 to about 7, about 5.5 to about 6.8, about 5.5 to about 6.6, about 5.5 to about 6.5, about 6.0 to about 7.5, about 6.0 to about 7.2, about 6.0 to about 7, about 6.0 to about 6.8, about 6.0 to about 6.6, about 6.0 to about 6.5, about 6.4 to about 7.5, about 6.4 to about 7.2, about 6.4 to about 7, about 6.4 to about 6.8, about 6.4 to about 6.6, about 6.4 to about 6.5, about 5.2, about 5.3, about 5.4, or about 6.5.

The buffer solution may be included at a concentration of about 0.1 mM to about 50 mM, about 0.1 mM to about 40 mM, about 0.1 mM to about 30 mM, about 0.1 mM to about 20 mM, about 0.1 mM to about 12 mM, about 0.1 mM to about 10 mM, about 0.1 mM to about 7.8 mM, about 0.1 mM to about 6 mM, about 0.1 mM to about 4 mM, about 0.1 mM to about 3 mM, about 0.1 mM to about 2.7 mM, about 1 mM to about 50 mM, about 1 mM to about 40 mM, about 1 mM to about 30 mM, about 1 mM to about 20 mM, about 1 mM to about 12 mM, about 1 mM to about 10 mM, about 1 mM to about 7.8 mM, about 1 mM to about 6 mM, about 1 mM to about 4 mM, about 1 mM to about 3 mM, about 1 mM to about 2.7 mM, about 2 mM to about 50 mM, about 2 mM to about 40 mM, about 2 mM to about 30 mM, about 2 mM to about 20 mM, about 2 mM to about 12 mM, about 2 mM to about 10 mM, about 2 mM to about 7.8 mM, about 2 mM to about 6 mM, about 2 mM to about 2.7 mM, about 2.5 mM to about 50 mM, about 2.5 mM to about 40 mM, about 2.5 mM to about 30 mM, about 2.5 mM to about 20 mM, about 2.5 mM to about 12 mM, about 2.5 mM to about 10 mM, about 2.5 mM to about 7.8 mM, about 2.5 mM to about 6 mM, about 2.7 mM to about 50 mM, about 2.7 mM to about 40 mM, about 2.7 mM to about 30 mM, about 2.7 mM to about 20 mM, about 2.7 mM to about 12 mM, about 2.7 mM to about 10 mM, about 2.7 mM to about 7.8 mM, about 2.7 mM to about 6 mM, about 2.7 mM to about 5.4 mM, about 3 mM to about 50 mM, about 3 mM to about 40 mM, about 3 mM to about 30 mM, about 3 mM to about 20 mM, about 3 mM to about 12 mM, about 3 mM to about 10 mM, about 3 mM to about 7.8 mM, about 3 mM to about 6 mM, about 3 mM to about 5.4 mM, about 1 mM, about 2.5 mM, about 2.6 mM, about 2.7 mM, about 2.8 mM, about 2.9 mM, about 4.8 mM, about 4.9 mM, about 5 mM, about 5.1 mM, about 5.2 mM, about 5.3 mM, about 5.4 mM, about 7.2 mM, about 7.3 mM, about 7.4 mM, about 7.5 mM, about 7.6 mM, about 7.7 mM, about 7.8 mM, about 9.8 mM, or about 10 mM, based on the total pharmaceutical composition.

In an embodiment, the buffer solution may include citrate, and may be included at a concentration of about 0.1 mM to about 50 mM, about 0.1 mM to about 40 mM, about 0.1 mM to about 30 mM, about 0.1 mM to about 20 mM, about 0.1 mM to about 12 mM, about 0.1 mM to about 10 mM, about 1 mM to about 50 mM, about 1 mM to about 40 mM, about 1 mM to about 30 mM, about 1 mM to about 20 mM, about 1 mM to about 12 mM, about 1 mM to about 10 mM, about 2 mM to about 50 mM, about 2 mM to about 40 mM, about 2 mM to about 30 mM, about 2 mM to about 20 mM, about 2 mM to about 12 mM, about 2 mM to about 10 mM, about 2.5 mM to about 50 mM, about 2.5 mM to about 40 mM, about 2.5 mM to about 30 mM, about 2.5 mM to about 20 mM, about 2.5 mM to about 12 mM, about 2.5 mM to about 10 mM, about 3 mM to about 50 mM, about 3 mM to about 40 mM, about 3 mM to about 30 mM, about 3 mM to about 20 mM, about 3 mM to about 12 mM, about 3 mM to about 10 mM, about 9.8 mM, or about 10 mM, based on the total pharmaceutical composition.

In another embodiment, the buffer solution may include one or more (e.g., one, two, or three) selected from the group consisting of phosphate, succinate, and acetate.

In an embodiment, the buffer solution may include phosphate. In another embodiment, the buffer solution may include phosphate, and may be free of or may be substantially free of citrate. In another embodiment, the buffer solution may include (1) phosphate and (2) succinate or acetate. In another embodiment, the buffer solution may include (1) phosphate and (2) succinate or acetate, and may be free of or may be substantially free of citrate.

A concentration of the phosphate may be about 0.1 mM to about 10 mM, about 0.1 mM to about 7.8 mM, about 0.1 mM to about 6 mM, about 0.1 mM to about 4 mM, about 0.1 mM to about 3 mM, about 0.1 mM to about 2.7 mM, about 1 mM to about 10 mM, about 1 mM to about 7.8 mM, about 1 mM to about 6 mM, about 1 mM to about 4 mM, about 1 mM to about 3 mM, about 1 mM to about 2.7 mM, about 2 mM to about 10 mM, about 2 mM to about 7.8 mM, about 2 mM to about 6 mM, about 2 mM to about 2.7 mM, about 2.5 mM to about 10 mM, about 2.5 mM to about 7.8 mM, about 2.5 mM to about 6 mM, about 2.5 mM to about 2.7 mM, about 1 mM, about 2.5 mM, about 2.6 mM, about 2.7 mM, about 2.8 mM, or about 2.9 mM, based on the total pharmaceutical composition.

A concentration of the succinate and/or acetate may be about 0.1 mM to about 20 mM, about 0.1 mM to about 12 mM, about 0.1 mM to about 10 mM, about 0.1 mM to about 7.8 mM, about 0.1 mM to about 6 mM, about 0.1 mM to about 5.4 mM, about 1 mM to about 20 mM, about 1 mM to about 12 mM, about 1 mM to about 10 mM, about 1 mM to about 7.8 mM, about 1 mM to about 6 mM, about 1 mM to about 5.4 mM, about 2 mM to about 20 mM, about 2 mM to about 12 mM, about 2 mM to about 10 mM, about 2 mM to about 7.8 mM, about 2 mM to about 6 mM, about 2 mM to about 5.4 mM, about 2.5 mM to about 20 mM, about 2.5 mM to about 12 mM, about 2.5 mM to about 10 mM, about 2.5 mM to about 7.8 mM, about 2.5 mM to about 6 mM, about 2.5 mM to about 5.4 mM, about 3 mM to about 20 mM, about 3 mM to about 12 mM, about 3 mM to about 10 mM, about 3 mM to about 7.8 mM, about 3 mM to about 6 mM, about 3 mM to about 5.4 mM, about 4.8 mM, about 4.9 mM, about 5 mM, about 5.1 mM, about 5.2 mM, about 5.3 mM, or about 5.4 mM, based on the total pharmaceutical composition.

(5) Polyol

In the present disclosure, the polyol may be one or more selected from the group consisting of sugars and sugar alcohols. For example, the polyol may be one or more selected from the group consisting of sorbitol, mannitol, meglumine, trehalose, sucrose, maltose, lactose, glucose, xylitol, arabitol, erythritol, lactitol, maltitol, inositol, etc. In the present disclosure, the polyol, e.g., sorbitol may be used as a tonicity agent or a stabilizer. In another embodiment, the polyol, e.g., mannitol may be used as a tonicity agent or a stabilizer.

In an embodiment, the polyol may be sorbitol, mannitol, or a combination thereof. In an embodiment, the polyol may include sorbitol, and may be free of or may be substantially free of one or more selected from sugars and sugar alcohols other than sorbitol, or all of them.

In another embodiment, the polyol may include mannitol, and may be free of or may be substantially free of one or more selected from sugars and sugar alcohols other than mannitol, or all of them.

To more improve the protein stability in the pharmaceutical composition provided in the present disclosure or to be served as a tonicity agent, a concentration of the polyol may be about 0.01 %(w/v) to about 10 %(w/v), about 0.01 %(w/v) to about 8 %(w/v), about 0.01 %(w/v) to about 6 %(w/v), about 0.01 %(w/v) to about 4 %(w/v), about 0.01 %(w/v) to about 3.5 %(w/v), about 0.01 %(w/v) to about 3.3 %(w/v), about 0.01 %(w/v) to about 2.6 %(w/v), about 0.01 %(w/v) to about 2 %(w/v), about 0.01 %(w/v) to about 1 %(w/v), about 0.05 %(w/v) to about 10 %(w/v), about 0.05 %(w/v) to about 8 %(w/v), about 0.05 %(w/v) to about 6 %(w/v), about 0.05 %(w/v) to about 4 %(w/v), about 0.05 %(w/v) to about 3.5 %(w/v), about 0.05 %(w/v) to about 3.3 %(w/v), about 0.05 %(w/v) to about 2.6 %(w/v), about 0.05 %(w/v) to about 2 %(w/v), about 0.05 %(w/v) to about 1 %(w/v), about 0.1 %(w/v) to about 10 %(w/v), about 0.1 %(w/v) to about 8 %(w/v), about 0.1 %(w/v) to about 6 %(w/v), about 0.1 %(w/v) to about 4 %(w/v), about 0.1 %(w/v) to about 3.5 %(w/v), about 0.01 %(w/v) to about 3.3 %(w/v), about 0.01 %(w/v) to about 2.6 %(w/v), about 0.1 %(w/v) to about 2 %(w/v), about 0.1 %(w/v) to about 1 %(w/v), about 1 %(w/v) to about 10 %(w/v), about 1 %(w/v) to about 8 %(w/v), about 1 %(w/v) to about 6 %(w/v), about 1 %(w/v) to about 4 %(w/v), about 1 %(w/v) to about 3.5 %(w/v), about 1 %(w/v) to about 3.3 %(w/v), about 1 %(w/v) to about 2.6 %(w/v), about 1 %(w/v) to about 2 %(w/v), about 1.5 %(w/v) to about 10 %(w/v), about 1.5 %(w/v) to about 8 %(w/v), about 1.5 %(w/v) to about 6 %(w/v), about 1.5 %(w/v) to about 4 %(w/v), about 1.5 %(w/v) to about 3.5 %(w/v), about 1.5 %(w/v) to about 3.3 %(w/v), about 1.5 %(w/v) to about 2.6 %(w/v), about 2 %(w/v) to about 10 %(w/v), about 2 %(w/v) to about 8 %(w/v), about 2 %(w/v) to about 6 %(w/v), about 2 %(w/v) to about 4 %(w/v), about 2 %(w/v) to about 3.5 %(w/v), about 2 %(w/v) to about 3.3 %(w/v), about 2 %(w/v) to about 2.6 %(w/v), about 2.3 %(w/v) to about 10 %(w/v), about 2.3 %(w/v) to about 8 %(w/v), about 2.3 %(w/v) to about 6 %(w/v), about 2.3 %(w/v) to about 4 %(w/v), about 2.3 %(w/v) to about 3.5 %(w/v), about 2.3 %(w/v) to about 3.3 %(w/v), about 2.3 %(w/v) to about 2.6 %(w/v), about 2.5 %(w/v) to about 10 %(w/v), about 2.5 %(w/v) to about 8 %(w/v), about 2.5 %(w/v) to about 6 %(w/v), 2.5 %(w/v) to about 4 %(w/v), about 2.5 %(w/v) to about 3.5 %(w/v), about 2.5 %(w/v) to about 3.3 %(w/v), about 2.5 %(w/v) to about 3 %(w/v), about 2.3%(w/v), about 2.4%(w/v), about 2.5%(w/v), about 2.6%(w/v), about 2.7%(w/v), about 2.8%(w/v), about 2.9%(w/v), about 3%(w/v), about 3.1%(w/v), about 3.2%(w/v), or about 3.3%(w/v), based on the total pharmaceutical composition.

(6) pH

pH of the pharmaceutical composition provided in the present disclosure may be about 4 to about 8, and specifically, about 4.0 to about 7.5, about 4.0 to about 7, about 4.0 to about 6.5, about 4.0 to about 6, about 4.0 to about 5.5, about 4.5 to about 7.5, about 4.5 to about 7, about 4.5 to about 6.5, about 4.5 to about 6, about 4.5 to about 5.5, about 4.8 to about 7.5, about 4.8 to about 7, about 4.8 to about 6.5, about 4.8 to about 6, about 4.8 to about 5.6, about 5.0 to about 7.5, about 5.0 to about 7, about 5.0 to about 6.5, about 5.0 to about 6, about 5.0 to about 5.5, about 5.2 to about 7.5, about 5.2 to about 7.2, about 5.2 to about 7, about 5.2 to about 6.8, about 5.2 to about 6.6, about 5.2 to about 6.5, about 5.5 to about 7.5, about 5.5 to about 7.2, about 5.5 to about 7, about 5.5 to about 6.8, about 5.5 to about 6.6, about 5.5 to about 6.5, about 6.0 to about 7.5, about 6.0 to about 7.2, about 6.0 to about 7, about 6.0 to about 6.8, about 6.0 to about 6.6, about 6.0 to about 6.5, about 6.4 to about 7.5, about 6.4 to about 7.2, about 6.4 to about 7, about 6.4 to about 6.8, about 6.4 to about 6.6, about 6.4 to about 6.5, about 5.0, about 5.2, about 5.3, about 5.4, about 5.5 or about 6.5.

(7) Other additives

The pharmaceutical composition provided in the present disclosure may be free of or may be substantially free of one or more selected from the group consisting of ammonium salts, e.g., ammonium chloride, ammonium sulfate, ammonium carbonate, ammonium nitrate, etc. In another embodiment, the pharmaceutical composition provided in the present disclosure may be free of or may be substantially free of sodium chloride, sodium sulfate, potassium chloride, sodium hydroxide, potassium hydroxide, ethylenediaminetetraacetic acid (EDTA), or all of them.

In addition, the pharmaceutical composition may further include a pharmaceutically acceptable carrier, diluent, and/or excipient. The pharmaceutically acceptable carrier may be those commonly used, and may include one or more selected from the group consisting of lactose, dextrose, sucrose, sorbitol, mannitol, starch, acacia rubber, calcium phosphate, alginate, gelatin, calcium silicate, microcrystalline cellulose, polyvinylpyrrolidone, cellulose, water (e.g., purified water), physiological saline, syrup, methyl cellulose, methylhydroxybenzoate, propylhydroxybenzoate, talc, magnesium stearate, mineral oil, etc., but is not limited thereto.

(8) Physical properties

The pharmaceutical composition provided in the present disclosure may have excellent stability, as compared with a histidine-free composition, a composition including polysorbate 80 instead of polysorbate 20, or both of them.

In an embodiment, the histidine-free composition may be completely free of histidine (about 0 mM) or may be substantially free of histidine.

In an embodiment, the composition including polysorbate 80 instead of polysorbate 20 (by replacing polysorbate 20) may include polysorbate 80 in an amount of 0.01%(w/v) to 0.2%(w/v), 0.01%(w/v) to 0.15%(w/v), 0.01%(w/v) to 0.12%(w/v), 0.01%(w/v) to 0.1%(w/v), 0.04%(w/v) to 0.2%(w/v), 0.04%(w/v) to 0.15%(w/v), 0.04%(w/v) to 0.12%(w/v), 0.04%(w/v) to 0.1%(w/v), 0.08%(w/v) to 0.2%(w/v), 0.08%(w/v) to 0.15%(w/v), 0.08%(w/v) to 0.12%(w/v), or 0.08%(w/v) to 0.1%(w/v), based on the total pharmaceutical composition, but is not limited thereto.

Stability

The pharmaceutical composition provided in the present disclosure may have excellent stability. As used herein, "excellent stability" or "stably maintained" may mean that a structure, and/or physical, chemical, and/or biological properties of the protein in the composition are maintained during preservation (for example, low protein polymer formation rate, low protein aggregation rate, low protein degradation rate, high protein (drug) contents, low denaturation rates, low oxidation level of amino acids (e.g., methionine residue), etc., during preservation).

The pharmaceutical composition provided in the present disclosure may have one or more of the following characteristics:

(i) improvement in photostability, as compared with a histidine-free composition; and
(ii) improvement in thermal stability, freeze-thaw stability, or both thermal stability and freeze-thaw stability, as compared with a composition including polysorbate 80 instead of polysorbate 20.

The photostability may mean that a structure, and/or physical, chemical, and/or biological properties of the protein in the composition are maintained under photostress conditions (for example, low protein polymer formation rate, low protein aggregation rate, low protein degradation rate, high protein (drug) contents, low denaturation rates, low oxidation level of amino acids (e.g., methionine residue), etc., during preservation). In an embodiment, the photostability may be measured (or determined or examined or analyzed) by low oxidation level of amino acids (e.g., methionine residue) under light-exposed conditions.

In an embodiment, the improvement in photostability may mean that the oxidation level of the amino acid residue of the antibody under photostress conditions is lowered (antioxidation). In an embodiment, the amino acid residue may be one or more methionines present at heavy chain and/or light chain of the antibody (anti-TNFa antibody, e.g., adalimumab), for example, methionine at position 256 of the heavy chain of the antibody. The antioxidant effect on amino acid residues of the antibody in the pharmaceutical composition under photostress conditions may be attributed to the action of histidine included in the pharmaceutical composition.

In an embodiment, the photostress conditions may be those in accordance with the International Council for Harmonisation of Technical Requirements for Pharmaceuticals for Human Use (ICH) guideline, 'Q1B Stability Testing: Photostability Testing of New Drug Substances and Products' (see Reference Example 2; base, dark control, light-exposed, e.g., conditions of 1.2 million lux hours or more and 200 watt hours/square meter (Wh/m$^2$) or more.

In an embodiment, with regard to the pharmaceutical composition provided in the present disclosure, an oxidation rate (%Met$_{256}$) of the methionine residue at position 256 of a heavy chain of the antibody, as measured under the light-exposed conditions, may be about 64% or less, about 63% or less, about 62% or less, about 61% or less, about 60% or less, about 59% or less, about 58% or less, about 57% or less, about 56% or less, about 55% or less, about 54% or less, about 53% or less, about 52% or less, about 51% or less, about 50% or less, about 49% or less, about 48% or less, about 47% or less, about 46% or less, about 45% or less, about 44% or less, about 43% or less, about 42% or less, or about 41% or less (the lower limit value is a value selected from the range of more than 0% and 41% or less, e.g., about 0%, about 5%, about 10%, about 15%, about 20%, about 25%, about 30%, about 35%, or about 40%, but is not limited thereto).

In another embodiment, with regard to the pharmaceutical composition provided in the present disclosure, the oxidation rate ($\%Met_{256}$) of the methionine residue at position 256 of a heavy chain of the antibody, as measured under the light-exposed conditions, may exhibit about 10% or more, about 11% or more, about 12% or more, about 13% or more, about 14% or more, about 15% or more, about 16% or more, about 17% or more, about 18% or more, about 19% or more, about 20% or more, about 21% or more, about 22% or more, about 23% or more, about 24% or more or about 25% or more reduction (the upper limit value is a value selected from the range of 25% to 100%, e.g., about 26%, about 27%, about 28%, about 29%, about 30%, about 35%, about 40%, about 45%, or about 50%, but is not limited thereto), as compared with $\%Met_{256}$ of the histidine-free composition, as measured under the light-exposed conditions.

In another embodiment, with regard to the pharmaceutical composition provided in the present disclosure, a difference ($\Delta\%Met_{256}$) between the oxidation rate ($\%Met_{256}$) of the methionine residue at position 256 of a heavy chain of the antibody, as measured under the light-exposed conditions, and $\%Met_{256}$ of a base group (without light exposure, $5\pm3$ °C) or a dark control (treated in a light blocking state under the same light-exposed conditions (e.g., blocking with foil)) may be 65% or less, 60% or less, 55% or less, 54% or less, 53% or less, 50% or less, 45% or less, 40% or less, 35% or less, 30% or less, 29% or less, 28% or less, 27% or less, 26% or less, 25% or less, or 24% or less (the lower limit value is a value selected from the range of more than 0% and 24% or less, e.g., about 0%, about 5%, about 10%, about 15%, or about 20%, but is not limited thereto).

In another embodiment, with regard to the pharmaceutical composition provided in the present disclosure, a difference ($\Delta\%Met_{256}$) between the oxidation rate ($\%Met_{256}$) of the methionine residue at position 256 of a heavy chain of the antibody, as measured under the light-exposed conditions, and $\%Met_{256}$ of the base group (without light exposure, $5\pm3$ °C) or the dark control (treated in a light blocking state under the same light-exposed conditions (e.g., blocking with foil)) may exhibit about 10% or more, about 11% or more, about 12% or more, about 13% or more, about 14% or more, about 15% or more, about 16% or more, about 17% or more, about 18% or more, about 19% or more, about 20% or more, about 21% or more, about 22% or more, about 23% or more, about 24% or more or about 25% or more (the upper limit value is a value selected from the range of 25% to 100%, e.g., about 26%, about 27%, about 28%, about 29%, about 30%, about 35%, about 40%, about 45%, or about 50%, but is not limited thereto) reduction, as compared with that of the histidine-free composition.

In an embodiment, the improvement in photostability may refer to reduction (anti-aggregation) of the aggregation rate of the antibody in the composition under photostress conditions. Such an effect of reduction (anti-aggregation) of the aggregation rate of the antibody under photostress conditions may be attributed to the action of histidine included in the pharmaceutical composition.

The protein aggregation rate may refer to a content ratio of aggregate in the composition, and the 'aggregate' may refer to a polymer product (high molecular weight; HMW) resulting from aggregation of the anti-TNFa antibody proteins which are included in the composition of the present disclosure.

In the photostability, the protein aggregation rate may be represented as an aggregate content (%HMW or HMW%) or a change or difference ($\Delta\%HMW$ or $\Delta HMW\%$) in the aggregate content in the composition, as measured under the photostress conditions.

In an embodiment, with regard to the pharmaceutical composition provided in the present disclosure, the aggregate content (%HMW)(e.g., measured by SE-HPLC) in the composition, as measured under the light-exposed conditions, may be about 15% or less, about 14% or less, about 13% or less, about 12% or less, about 11% or less, about 10% or less, about 9.5% or less, about 9% or less, or about 8.5% or less (the lower limit value is a value selected from the range of more than 0% and 8.5% or less, e.g., about 0%, about 0.1%, about 0.5%, about 1%, or about 2%, but is not limited thereto).

In an embodiment, with regard to the pharmaceutical composition provided in the present disclosure, the aggregate content (%HMW)(e.g., measured by SE-HPLC) in the composition, as measured under the light-exposed conditions, may exhibit about 4% or more, about 5% or more, about 6% or more, about 7% or more, about 8% or more, about 9% or more, or about 10% or more (the upper limit value is a value selected from the range of 10% to 100%, e.g., about 11%, about 15%, about 20%, about 25%, about 30%, about 35%, about 40%, about 45%, or about 50%, but is not limited thereto) reduction, as compared with that of the histidine-free composition.

In an embodiment, with regard to the pharmaceutical composition provided in the present disclosure, a change or difference ($\Delta\%HMW$) in the aggregate content in the composition, as measured under the photostress conditions, i.e., a difference [$\Delta\%HMW = (\%HMW$ under the light-exposed conditions) - (%HMW under base or dark control conditions)] between the aggregation content under the light-exposed conditions and the aggregation content under base or dark control conditions may be about 15% or less, about 14% or less, about 13% or less, about 12% or less, about 11% or less, about 10% or less, about 9.5% or less, about 9% or less, about 8.8% or less, about 8.6% or less, about 8.5% or less, or about 8% or less (the lower limit value is a value selected from the range of more than 0% and 8% or less, e.g., about 0%, about 0.1%, about 0.5%, about 1%, or about 2%, but is not limited thereto).

In an embodiment, with regard to the pharmaceutical composition provided in the present disclosure, a change or difference [$\Delta$%HMW = (%HMW under the light-exposed conditions) - (%HMW under base or dark control conditions)] in the aggregate content in the composition, as measured under the photostress conditions, may exhibit about 3% or more, about 4% or more, about 5% or more, about 5.5% or more, about 6% or more, about 7% or more, about 8% or more, about 9% or more, about 10% or more, about 11% or more, or about 12% or more (the upper limit value is a value selected from the range of 12% to 100%, e.g., about 13%, about 15%, about 20%, about 25%, about 30%, about 35%, about 40%, about 45% or about 50%, but is not limited thereto) reduction, as compared with that of the histidine-free composition.

The thermal stability and/or freeze-thaw stability mean that a structure, and/or physical, chemical, and/or biological properties of the protein in the composition are maintained, when stored or preserved under heat stress conditions (e.g., at 40±2 °C for 0 week to 8 weeks or 0 week to 4 weeks (e.g., 0 week, 1 week, 2 weeks, 3 weeks, 4 weeks, or 8 weeks)) and/or under freeze/thaw conditions (e.g., 3~5 cycles of 'frozen at - 70 °C±10 °C for 18 hours or longer' and 'thawed at room temperature (25 °C) for 1 hour or longer') (for example, low protein polymer formation rate, low protein aggregation rate, low protein degradation rate, high protein (drug) contents, low denaturation rates, low oxidation level of amino acids (e.g., methionine residue), etc. at any one or more given time points during a period of storage or preservation).

In an embodiment, the thermal stability and/or the freeze-thaw stability may be measured (or determined or examined or analyzed) by a protein aggregation rate, a protein denaturation rate, or a combination thereof, which is measured under heat stress conditions and/or freeze-thaw conditions. In an embodiment, the improvement in the thermal stability and/or the freeze-thaw stability may refer to reduction of the protein aggregation rate, the protein denaturation rate, or both of them under heat stress conditions and/or freeze-thaw conditions.

With regard to the thermal stability and/or the freeze-thaw stability, the protein aggregation rate may be represented as:

- an aggregate content (%HMW or HMW%) in the composition at any given time point during a period of preservation (or storage) including the initial time of preservation (or storage),
- a change or difference ($\Delta$%HMW) of the aggregate content in the composition at any given time point during a period of preservation (or storage) including the initial time of preservation (or storage),
- an aggregate content (%HMW or HMW%) in the composition after 5 cycles of freeze-thaw under -70±10 °C/25 °C conditions, and/or
- a change or difference ($\Delta$%HMW) of the aggregate content in the composition before and after 5 cycles of freeze-thaw under -70±10 °C/25 °C conditions

.

As used herein, the term 'denaturation rate' refers to a degree of acidity (acidic variant content; %Acidic or Acidic%) or a degree of basicity (basic variant content; %Basic or Basic%) of the anti-TNFa antibody protein included in the composition of the present disclosure, relative to its original property.

In an embodiment, the protein denaturation rate may be represented as:

- an acidic variant content (%Acidic or Acidic%) in the composition at any given time point during a period of preservation (or storage) including the initial time of preservation (or storage),
- a change or difference ($\Delta$%Acidic) of the acidic variant content in the composition at any given time point during a period of preservation (or storage) including the initial time of preservation (or storage),
- an acidic variant content (%Acidic or Acidic%) in the composition after 5 cycles of freeze-thaw under -70±10 °C/25 °C conditions, and/or
- a change or difference ($\Delta$%Acidic = (FT5 %Acidic) - (Initial %Acidic)) of the acidic variant content in the composition before and after 5 cycles of freeze-thaw under -70±10 °C/25 °C conditions

.

In an embodiment, with regard to the pharmaceutical composition provided in the present disclosure, the aggregate content (%HMW) in the composition (e.g., measured by SE-HPLC) may be about 1.0% or less, about 0.97% or less, about 0.94% or less, about 0.91% or less, about 0.87% or less, about 0.84% or less, about 0.81% or less, about 0.79% or less, about 0.76% or less, about 0.72% or less, about 0.70% or less, about 0.69% or less or about 0.68% or less, and the lower limit value thereof may be a value of more than 0, e.g., about 0.0001%, about 0.0005%, about 0.001%, about 0.004%, about 0.007%, or about 0.01% at any given time point (e.g., 0 week, 1 week, 2 weeks, 4 weeks, or 8 weeks) during a period of 0 week to 8 weeks, 0 week to 4 weeks, or 0 week to 2 weeks (a period from start of preservation to end of preservation) or over the entire period at a temperature of $40 \pm 2$ °C.

In an embodiment, with regard to the pharmaceutical composition provided in the present disclosure, the aggregate content (%HMW) in the composition (e.g., measured by SE-HPLC) may exhibit about 0.005% or more, about 0.01% or more, about 0.015% or more, about 0.02% or more, about 0.04% or more, about 0.06% or more, or about 0.08% or more (the upper limit value is a value selected from the range of 0.08% to 100%, e.g., about 0.1%, about 1%, about 1.5%, about 5%, about 10%, about 20%, about 30%, about 40% or about 50%, but is not limited thereto) reduction, as compared with that of the composition including polysorbate 80 instead of polysorbate 20 (e.g., at the same concentration), at any given time point (e.g., 0 week, 1 week, 2 weeks, 4 weeks, or 8 weeks) during a period of 0 week to 8 weeks, 0 week to 4 weeks, or 0 week to 2 weeks (a period from start of preservation to end of preservation) or over the entire period at a temperature of $40 \pm 2$ °C.

In an embodiment, with regard to the pharmaceutical composition provided in the present disclosure, the aggregate content (%HMW) in the composition (e.g., measured by SE-HPLC) may exhibit about 0.1% or more, about 0.15% or more, about 0.2% or more, about 0.25% or more, about 0.3% or more, about 0.35% or more, about 0.4% or more, about 0.45% or more, about 0.5% or more, or about 0.55% or more (the upper limit value is a value selected from the range of 0.55% to 100%, e.g., about 0.6%, about 1%, about 1.5%, about 5%, about 10%, about 20%, about 30%, about 40% or about 50%, but is not limited thereto) reduction, as compared with that of the composition being free of histidine and including polysorbate 80 instead of polysorbate 20, at any given time point (e.g., 0 week, 1 week, 2 weeks, 4 weeks, or 8 weeks) during a period of 0 week to 8 weeks, 0 week to 4 weeks, or 0 week to 2 weeks (a period from start of preservation to end of preservation) or over the entire period at a temperature of $40 \pm 2$ °C.

In an embodiment, with regard to the pharmaceutical composition provided in the present disclosure, a difference ($\Delta$%HMW) in the aggregate content in the composition may be about 1.0% or less, about 0.97% or less, about 0.94% or less, about 0.91% or less, about 0.87% or less, about 0.84% or less, about 0.81% or less, about 0.79% or less, about 0.76% or less, about 0.72% or less, about 0.70% or less, about 0.68% or less, about 0.66% or less, about 0.64% or less, about 0.62% or less, about 0.60% or less, about 0.58% or less, about 0.56% or less, about 0.54% or less, about 0.52% or less, about 0.50% or less, about 0.48% or less, about 0.46% or less, about 0.44% or less, about 0.42% or less, about 0.40% or less, about 0.38% or less, about 0.36% or less, about 0.34% or less, about 0.32% or less, about 0.30% or less, about 0.28% or less, or about 0.26% or less, and the lower limit value thereof may be a value of more than 0, e.g., about 0.0001%, about 0.0005%, about 0.001%, about 0.005%, about 0.01%, about 0.05%, or about 0.1%, during a period of 0 week to 8 weeks, 0 week to 4 weeks, or 0 week to 2 weeks (e.g. 0 week, 1 week, 2 weeks, 4 weeks, or 8 weeks) at a temperature of $40 \pm 2$ °C.

In an embodiment, with regard to the pharmaceutical composition provided in the present disclosure, a difference ($\Delta$%HMW) in the aggregate content in the composition may exhibit about 0.02% or more, about 0.04% or more, about 0.06% or more, about 0.08% or more, about 0.1% or more, about 0.15% or more, about 0.2% or more, about 0.25% or more, about 0.3% or more, about 0.35% or more, or about 0.38% or more (the upper limit value is a value selected from the range of 0.38% to 100%, e.g., about 0.4%, about 1%, about 1.5%, about 5%, about 10%, about 20%, about 30%, about 40% or about 50%, but is not limited thereto) reduction, as compared with that of the composition being free of histidine and including polysorbate 80 instead of polysorbate 20, during a period of 0 week to 8 weeks, 0 week to 4 weeks, or 0 week to 2 weeks (e.g. 0 week, 1 week, 2 weeks, 4 weeks, or 8 weeks) at a temperature of $40 \pm 2$ °C.

In an embodiment, with regard to the pharmaceutical composition provided in the present disclosure, the acidic variant content (%Acidic) in the composition (e.g., measured by CEX-HPLC or icIEF) may be about 45% or less, about 43% or less, about 40% or less, about 38% or less, about 36% or less, about 34% or less, about 32% or less, about 30% or less, about 28% or less, or about 26% or less, and the lower limit value thereof may be a value of more than 0, e.g., about 0.0001%, about 0.0005%, about 0.001%, about 0.005%, about 0.01%, about 0.05%, or about 0.1%, at any given time point (e.g., 0 week, 1 week, 2 weeks, 4 weeks, or 8 weeks) during a period of 0 week to 8 weeks, 0 week to 4 weeks, or 0 week to 2 weeks (a period from start of preservation to end of preservation) or over the entire period at a temperature of $40 \pm 2$ °C.

In an embodiment, with regard to the pharmaceutical composition provided in the present disclosure, the acidic variant content (%Acidic) in the composition (e.g., measured by CEX-HPLC or icIEF) may exhibit about 1% or more, about 2% or more, about 3% or more, about 4% or more, about 5% or more, about 6% or more, about 7% or more, about 8% or more, about 9% or more, or about 10% or more (the upper limit value is a value selected from the range of 10% to 100%, e.g., about 11%, about 15%, about 20%, about 25%, about 30%, about 35%, about 40%, about 45% or about 50%, but is not limited thereto) reduction, as compared with that of the composition being free of histidine and including polysorbate 80 instead of polysorbate 20, at any given time point (e.g., 0 week, 1 week, 2 weeks, 4 weeks, or 8 weeks) during a period of 0 week to 8 weeks, 0 week to 4 weeks, or 0 week to 2 weeks (a period from start of preservation to end of preservation) or over the entire period at a temperature of $40 \pm 2$ °C.

In an embodiment, with regard to the pharmaceutical composition provided in the present disclosure, a difference (Δ%Acidic) in the acidic variant content in the composition may be about 25% or less, about 24% or less, about 23% or less, about 22% or less, about 21% or less, about 20% or less, about 19% or less, about 18% or less, about 17.5% or less, about 17% or less, about 16.5% or less, about 16% or less, about 15.5% or less, about 15% or less, about 14.5% or less, about 14% or less, about 13.5% or less, about 13% or less, about 12.5% or less, about 12% or less, about 11.5% or less, about 11% or less, about 10.5% or less, about 10% or less, about 9.5% or less, or about 9% or less, and the lower limit value thereof may be a value of more than 0, e.g., about 0.0001%, about 0.0005%, about 0.001%, about 0.005%, about 0.01%, about 0.05%, or about 0.1%, during a period of 0 week to 8 weeks, 0 week to 4 weeks, or 0 week to 2 weeks (e.g., 0 week, 1 week, 2 weeks, 4 weeks, or 8 weeks) at a temperature of 40±2 °C.

In an embodiment, with regard to the pharmaceutical composition provided in the present disclosure, a difference (Δ%HMW) in the aggregate content in the composition may exhibit about 1% or more, about 1.5% or more, about 2% or more, about 3% or more, about 4% or more, about 5% or more, about 6% or more, about 7% or more, about 8% or more, about 9% or more, about 10% or more, about 11% or more, about 12% or more, or about 13% or more (the upper limit value is a value selected from the range of 13% to 100%, e.g., about 14%, about 15%, about 20%, about 25%, about 30%, about 35%, about 40%, about 45% or about 50%, but is not limited thereto) reduction, as compared with that of the composition being free of histidine and including polysorbate 80 instead of polysorbate 20, during a period of 0 week to 8 weeks, 0 week to 4 weeks, or 0 week to 2 weeks (e.g., 1 week, 2 weeks, 4 weeks, or 8 weeks) at a temperature of 40±2 °C.

In an embodiment, with regard to the pharmaceutical composition provided in the present disclosure, the aggregate content (%HMW) in the composition (e.g., measured by SE-HPLC) may be about 1.0% or less, about 0.97% or less, about 0.94% or less, about 0.91% or less, about 0.87% or less, about 0.84% or less, about 0.81% or less, about 0.79% or less, about 0.76% or less, about 0.72% or less, about 0.70% or less, about 0.68% or less, about 0.66% or less, about 0.64% or less, about 0.62% or less, about 0.60% or less, about 0.58% or less, about 0.57% or less, about 0.56% or less, about 0.55% or less, about 0.54% or less, about 0.53% or less, about 0.52% or less, about 0.51% or less, about 0.50% or less, about 0.49% or less, or about 0.48% or less, and the lower limit value thereof may be a value of more than 0, e.g., about 0.0001%, about 0.0005%, about 0.001%, about 0.005%, about 0.01%, about 0.05%, or about 0.1%, after 5 cycles of freeze-thaw under -70±10 °C/25 °C conditions.

In an embodiment, with regard to the pharmaceutical composition provided in the present disclosure, the aggregate content (%HMW) in the composition (e.g., measured by SE-HPLC) may exhibit about 0.005% or more, about 0.01% or more, about 0.015% or more, about 0.02% or more, about 0.04% or more, about 0.06% or more, about 0.08% or more, or about 0.1% or more (the upper limit value is a value selected from the range of 0.1% to 100%, e.g., about 0.2%, about 0.5%, about 1%, about 5%, about 10%, about 15%, about 40%, about 45% or about 50%, but is not limited thereto) reduction, as compared with that of the composition including polysorbate 80 instead of polysorbate 20 (e.g., at the same concentration), after 5 cycles of freeze-thaw under -70±10 °C/25 °C conditions.

In an embodiment, with regard to the pharmaceutical composition provided in the present disclosure, the acidic variant content (%Acidic) in the composition (e.g., measured by iclEF) may be about 27% or less, about 26.91% or less, or about 26.85% or less, and the lower limit value thereof may be a value of more than 0, e.g., about 0.0001%, about 0.0005%, about 0.001%, about 0.005%, about 0.01%, about 0.05%, or about 0.1%, after 5 cycles of freeze-thaw under -70±10 °C/25 °C conditions.

In an embodiment, with regard to the pharmaceutical composition provided in the present disclosure, the acidic variant content (%Acidic) in the composition (e.g., measured by iclEF) may exhibit about 0.005% or more, about 0.01% or more, about 0.1% or more, about 0.12% or more, about 0.15% or more, about 0.2% or more, about 0.5% or more, about 0.7% or more, or about 1% or more (the upper limit value is a value selected from the range of 1% to 100%, e.g., about 1.1%, about 1.5%, about 2%, about 5%, about 10%, about 15%, about 40%, about 45%, or about 50%, but is not limited thereto) reduction, as compared with that of the composition including polysorbate 80 instead of polysorbate 20 (e.g., at the same concentration), after 5 cycles of freeze-thaw under -70±10 °C/25 °C conditions.

In an embodiment, with regard to the pharmaceutical composition provided in the present disclosure, a change or difference (Δ%Acidic) in the acidic variant content in the composition (e.g., measured by iclEF) may be about 3% or less, about 2.6% or less, about 2.5% or less, about 2.45% or less, about 2% or less, about 1.9% or less, about 1.8% or less, about 1.7% or less, about 1.6% or less, about 1.5% or less, about 1.4% or less, about 1.3% or less, about 1.2% or less, about 1.1% or less, or about 1.0% or less, and the lower limit value thereof may be a value of more than 0, e.g., about 0.0001%, about 0.0005%, about 0.001%, about 0.005%, about 0.01%, about 0.05%, or about 0.1%, before and after 5 cycles of freeze-thaw under -70±10 °C/25 °C conditions.

In an embodiment, with regard to the pharmaceutical composition provided in the present disclosure, a change or difference (Δ%Acidic) in the acidic variant content in the composition may exhibit about 0.1% or more, about 0.2% or more, about 0.3% or more, about 0.4% or more, about 0.5% or more, about 0.6% or more, about 0.7% or more, about 0.8% or more, about 0.9% or more, about 1% or more, about 1.5% or more, or about 2% or more (the upper limit value is a value selected from the range of 2% to 100%, e.g., about 2.1%, about 2.5%, about 3%, about 5%, about 10%, about 15%, about 40%, about 45%, or about 50%, but is not limited thereto) reduction, as compared with that of the composition including polysorbate 80 instead of polysorbate 20 (e.g., at the same concentration), before and after 5 cycles of freeze-thaw under - 70±10 °C/25 °C conditions.

In an embodiment, the pharmaceutical composition provided in the present disclosure or the container including the same may maintain stability, when stored at a room temperature of 30±2 °C and relative humidity (RH) of 60±5% for about 28 days to about 35 days, about 28 days to about 32 days, about 28 days to about 30 days, about 28 days, or about 30 days, after preserved at a temperature of 5±3 °C for about 32 months to about 52 months, about 34 months to about 50 months, about 36 months to about 48 months, about 36 months, or about 48 months. In an embodiment, the roomtemperature storage stability may be measured (or determined or examined or analyzed) by one or more selected from the group consisting of clarity, color, pH, protein concentration, TNFα-binding activity, TNFα-neutralizing activity, purity, protein aggregation rate, charge variant, and the number of subvisible particles.

Specifically, the pharmaceutical composition provided in the present disclosure or the container including the same, when preserved at a temperature of 30±2 °C for 28 days to 35 days, specifically, 30 days, after being stored at a temperature of 5±3 °C for 32 months to 52 months, specifically, 36 months to 48 months, 36 months, or 48 months, may have one or more of the following characteristics:

turbidity may be about 30 NTU (nephelometric turbidity units) or less, about 25 NTU or less, about 22 NTU or less, about 20 NTU or less, about 19 NTU or less, about 18 NTU or less, or about 17 NTU or less, and the lower limit value thereof may be a value selected from 0 NTU to 10 NTU, but is not limited thereto.

TNFα-binding activity may be about 80% or more, about 85% or more, about 90% or more, about 91% or more, about 92% or more, about 93% or more, about 94% or more, about 95% or more, about 96% or more, about 97% or more, about 98% or more, about 100% or more, about 102% or more, about 104% or more, or about 106% or more, and the upper limit value thereof may be a value selected from the range of 107% to 125%, but is not limited thereto.

TNFα-neutralizing activity may be about 70% or more, about 75% or more, about 80% or more, about 85% or more, about 86% or more, about 90% or more, about 91% or more, about 92% or more, about 93% or more, about 94% or more, about 95% or more, about 96% or more, about 97% or more, about 98% or more, about 99% or more, about 100% or more, about 101% or more, about 102% or more, about 103% or more, about 104% or more, or about 105% or more, and the upper limit value thereof may be a value selected from the range of 105% to 133%, but is not limited thereto.

total purity may be about 90% or more, about 91% or more, about 92% or more, about 93% or more, about 94% or more, about 95% or more, about 96% or more, or about 97% or more, and the upper limit value thereof may be a value selected from the range of 97% to 100%, but is not limited thereto.

the aggregate content (%HMW) (e.g., measured by SE-HPLC) may be about 5% or less, about 4% or less, about 3% or less, about 2% or less, about 1% or less, or about 0.9% or less, and the lower limit value thereof may be a value of more than 0%, e.g., about 0%, about 0.001%, about 0.01%, about 0.1%, about 1%, but is not limited thereto.

the acidic variant content (%Acidic) (e.g., measured by iclEF) may be about 45% or less, about 43% or less, about 40% or less, about 38% or less, about 36% or less, about 34% or less, about 33% or less, and the lower limit value thereof may be a value of more than 0%, e.g., about 0%, about 1%, about 5%, about 10%, about 11%, about 12%, about 13%, about 14%, about 15% or about 16%, but is not limited thereto.

the main protein content (%Main) (e.g., measured by iclEF) may be about 50% or more, about 51% or more, about 52% or more, about 53% or more, about 54% or more, about 55% or more, about 55% or more, or about 56% or more, and the upper limit value thereof may be a value selected from the range of 50% to 100%, e.g., about 95%, about 93%, about 93%, about 90%, about 87%, about 85%, about 83%, about 80%, about 78%, about 75% or about 74%, but is not limited thereto.

the basic variant content (%basic) (e.g., measured by iclEF) may be about 20% or less, about 19% or less, about 18% or less, about 17% or less, about 16% or less, about 15% or less, or about 14% or less, and the lower limit value thereof may be a value of more than 0%, e.g., about 0%, about 1%, about 2%, about 3%, about 4%, about 5%, or about 6%, but is not limited thereto.

in 0.8 mL of the composition, the number of particles having an average particle diameter of 10 μm or more may be 6000 or less, 5000 or less, 4000 or less, 3000 or less, 2500 or less, 2000 or less, 1900 or less, 1800 or less, 1700 or less, 1600 or less, 1500 or less, 1400 or less, 1300 or less, 1200 or less, 1100 or less, or 1000 or less,

in 0.8 mL of the composition, the number of particles having an average particle diameter of 25 μm or more may be 600 or less, 500 or less, 400 or less, 300 or less, 200 or less, 100 or less, 70 or less, 50 or less, 40 or less, 30 or less, or 20 or less.

Osmotic pressure

The pharmaceutical composition provided in the present disclosure may be isotonic to a living body. Specifically, the liquid composition may have an osmotic pressure of about 200 mOsm/kg to about 400 mOsm/kg, about 250 mOsm/kg to about 400 mOsm/kg, about 200 mOsm/kg to about 350 mOsm/kg, about 250 mOsm/kg to about 350 mOsm/kg, about 200 to about 300 mOsm/kg or about 250 to about 300 mOsm/kg. Meanwhile, such an osmotic pressure may be controlled by polyol and/or amino acid, etc.

(9) Device, Kit, or Container

In an embodiment, provided is a device, a kit, or a container, each including the pharmaceutical composition provided in the present disclosure. The device, kit, or container may be mainly used for parenteral administration (e.g., subcutaneous administration), and may be in the form of a pharmaceutical composition-filled vial, pack, and syringe (e.g., pre-filled syringe; PFS); needle size: 20 G to 40 G, e.g., 25 G, 26 G, 27 G, 28 G, 29 G, or 30 G), pre-filled pen (PFP), etc., and the filled pharmaceutical composition may include one or more unit dosage forms, based on the content of the anti-TNFa antibody (e.g., adalimumab) which is an active ingredient.

In an embodiment, the pharmaceutical composition provided in the present disclosure may be, if desired, included in a vial, a pack, or a dispenser device that may include one or more unit dosage forms including the active ingredient. In an embodiment, the dispenser device may include a syringe containing a single dose of a liquid formulation of the pharmaceutical composition, which is prepared for injection. The syringe may be accompanied with an instruction for administration. In another embodiment, the device may be selected from Inject-ease®, Genject®, syringe pen (e.g., GenPen®), needleless device (e.g., MediJector®, BioJector®, etc.), but is limited thereto.

In the device, kit, or container including the pharmaceutical composition provided in the present disclosure, the included pharmaceutical composition may have stability against temperature changes. In an embodiment, the temperature changes mean that once or more, e.g., 3 cycles of exposure to a low temperature condition (e.g., -5±3 °C) following exposure to a high temperature condition (e.g., 30±2 °C) are performed. In an embodiment, the stability against temperature changes may be measured (or determined or examined or analyzed) by one or more selected from the group consisting of TNFα binding activity, TNFα neutralizing activity, the number of subvisible particles, oxidation of amino acid residues, clarity, pH, protein concentration, protein aggregation rate, purity, charge variants, and endotoxin.

In one specific embodiment, the pharmaceutical composition included in the device, kit, or container, after a total of 3 cycles, each cycle consisting of exposure to high temperature conditions of 30±2 °C (with 65±5% RH) for 48 hours, followed by exposure to low temperature conditions of -5±3 °C for 48 hours (exposure to high temperature conditions for a total of 144 hours and exposure to low temperature conditions for a total of 144 hours), may have one or more selected from the following characteristics:

TNFα-binding activity may be about 90% or more, about 91% or more, about 92% or more, about 93% or more, about 94% or more, about 95% or more, about 96% or more, about 97% or more, or about 98% or more (the upper limit value may be a value selected from the range of 100% to 125%),
TNFα-neutralizing activity may be about 90% or more, about 91% or more, about 92% or more, about 93% or more, about 94% or more, about 95% or more, about 96% or more, about 97% or more, about 98% or more, about 99% or more, about 100% or more, about 101% or more, about 102% or more, about 103% or more, about 104% or more, or about 105% or more (the upper limit value may be a value selected from the range of 100% to 133%),
the oxidation rate of methionine at position 34 of a heavy chain of the antibody may be about 1.5% or less, about 1.4% or less, about 1.3% or less, about 1.2% or less, about 1.1% or less, about 1.0% or less, about 0.9% or less, about 0.8% or less, about 0.7% or less, or about 0.6% or less (the lower limit value may be a value of more than 0, e.g., about 0.0001%, about 0.0005%, about 0.001%, about 0.005%, about 0.01%, about 0.05%, or about 0.1%),
the oxidation rate of methionine at position 83 of a heavy chain of the antibody may be about 1.3% or less, about 1.2% or less, about 1.1% or less, about 1.0% or less, about 0.9% or less, about 0.8% or less, about 0.7% or less, about 0.6% or less, about 0.5% or less, or about 0.4% or less (the lower limit value may be a value of more than 0, e.g., about 0.0001%, about 0.0005%, about 0.001%, about 0.005%, about 0.01%, about 0.05%, or about 0.1%),
the oxidation rate of methionine at position 256 of a heavy chain of the antibody may be about 6.0% or less, about 5.7% or less, about 5.5% or less, about 5.4% or less, about 5.3% or less, about 5.2% or less, about 5.1% or less, about 5.0% or less, about 4.9% or less, about 4.8% or less, or about 4.9% or less (the lower limit value may be a value of more than 0, e.g., about 0.01%, about 0.05%, about 0.1%, about 0.5%, or about 1%),
the oxidation rate of methionine at position 432 of a heavy chain of the antibody may be about 0.5% or less, about 0.4% or less, about 0.3% or less, about 0.2% or less, about 0.1% or less, about 0.05% or less, or may not be detected (methionine at position 432 is not oxidized at a detectable level) (the lower limit value may be selected from values including 0),

the oxidation rate of methionine at position 4 of a light chain of the antibody may be about 1.5% or less, about 1.3% or less, about 1.2% or less, about 1.1% or less, about 1.0% or less, about 0.9% or less, about 0.8% or less, about 0.7% or less, about 0.6% or less, or about 0.5% or less (the lower limit value may be a value of more than 0, e.g., about 0.0001%, about 0.0005%, about 0.001%, about 0.005%, about 0.01%, about 0.05%, or about 0.1%),

turbidity may be 25 NTU(nephelometric turbidity units) or less, 22 NTU or less, 20 NTU or less, 19 NTU or less, 18 NTU or less, or 17 NTU or less (the lower limit value may be selected from 0 NTU to 10 NTU),

total purity may be 90% or more, 91% or more, 92% or more, 93% or more, 94% or more, 95% or more, or 96% or more,

in 0.8 mL of the composition, the number of particles having an average particle diameter of 10 $\mu$m or more may be 5000 or less, 4000 or less, 3000 or less, 2500 or less, 2000 or less, 1900 or less, 1800 or less, 1700 or less, 1600 or less, 1500 or less,

in 0.8 mL of the composition, the number of particles having an average particle diameter of 25 $\mu$m or more may be 500 or less, 400 or less, 300 or less, 200 or less, 100 or less, 70 or less, 50 or less, 40 or less, 30 or less, or 20 or less, and

an endotoxin content may be 10 EU/mL or less, 9 EU/mL or less, 8 EU/mL or less, 7 EU/mL or less, 6 EU/mL or less, 5.5 EU/mL or less, or 5 EU/mL or less.

(13) Treatment of disease

In an embodiment, provided is a pharmaceutical composition for treating a disease, the pharmaceutical composition including the above-described pharmaceutical composition or the device including the same. In another embodiment, provided is a method of treating a disease, the method including administering a pharmaceutically effective amount of the pharmaceutical composition to a subject in need of administration of the anti-TNFa antibody, e.g., adalimumab. The treatment method may further include identifying the subject in need of administration of the anti-TNFa antibody, e.g., adalimumab, prior to the administering.

The disease may be selected from diseases on which a therapeutic effect may be obtained by administering the anti-TNFa antibody, e.g., adalimumab. The disease may be an immune-related disease, for example, may be selected from the group consisting of arthritis (e.g., rheumatoid arthritis, psoriatic arthritis, idiopathic arthritis, enthesitis-related arthritis, etc.), axial spondyloarthritis (e.g., ankylosing spondylitis, severe axial spondyloarthritis other than ankylosing spondylitis, etc.), Crohn's disease (e.g., adult (18 years or older) Crohn's disease, juvenile (6-17 years old) Crohn's disease, etc.), psoriasis (e.g., adult (18 years or older), or juvenile (4-17 years old) plaque psoriasis), ulcerative colitis, Behcet's enteritis, hidradenitis suppurativa, uveitis (e.g., non-infectious intermediate uveitis, posterior uveitis, and panuveitis, etc.).

The subject who needs the administration of the anti-TNFa antibody, e.g., adalimumab, may be a subject having a disease or a disorder that may be significantly treated (removal, reduction, relief, or alleviation of symptoms) by administering the anti-TNF$\alpha$ antibody, e.g., adalimumab. The disease or disorder may be selected from the above-described diseases.

(14) Administration Route, Administration Dose, and Formulation

The pharmaceutical composition provided in the present disclosure may be administered via a parenteral route. Parenteral administration (for example, injection) may be performed via an intravenous route or a subcutaneous route. Parenteral administration may be a bolus injection or a continuous injection.

The pharmaceutical composition provided in the present disclosure may be formulated into a form suitable for the above administration route. In an embodiment, the pharmaceutical composition may be formulated into an injection agent, or an injectable ready-to-use form, but is not limited thereto.

In another embodiment, the pharmaceutical composition may be formulated such that the entire amount or a pharmaceutically effective amount of the anti-TNFa antibody, e.g., adalimumab, may be included in a single dosage form or allocated into two or more (e.g., 2, 3, 4, 5, 6, 7, 8, 9, or 10) dosage forms. The formulated composition may be included in the above-described device as a unit dosage form.

In still another embodiment, the pharmaceutical composition may be formulated into a dosage form that allows the anti-TNFa antibody, e.g., adalimumab included in therein to be administered into a body all at once (e.g., within 1 minute, 30 seconds, 20 seconds, or 10 seconds) or slowly over 5 minutes or longer, 10 minutes or longer, 30 minutes or longer, 60 minutes or longer, 90 minutes or longer, 120 minutes or longer, 150 minutes or longer, 180 minutes or longer, 210 minutes or longer, or 240 minutes or longer, but is not limited thereto.

The subject to whom the pharmaceutical composition is administered may be selected from mammals including primates (humans, etc.), rodents (mice, rats, guinea pigs, hamsters, rabbits, etc.), cats, dogs, pigs, cow, horses, etc.

The pharmaceutically effective amount of the pharmaceutical composition provided in the present disclosure or the anti-TNFa antibody (e.g., adalimumab) included in the pharmaceutical composition may refer to an amount or a dose in the composition that may exhibit a desired pharmacological effect, for example, removal, reduction, relief, or alleviation of symptoms. The pharmaceutically effective amount may be determined depending on various factors including a formulation method, an administration manner, a patient's age, body weight, gender, illness state (severity of symptom), diets, administration time, administration interval, administration routes, excretion rate, responsiveness, previous therapy, clinical history, etc. The doses may be determined according to the prescription of the attending physician. The pharmaceutically effective amount may be administered once or in a series of administrations of twice or more by allocating.

In an embodiment, the pharmaceutical composition may be administered once to three times per a week over three weeks or more at such a dose as to include the desired effective amount, e.g., about 200 mg or less, about 150 mg or less, about 100 mg, about 50 mg, or 40 mg of the anti-TNFa antibody (e.g., adalimumab) (e.g., when the effective amount of the antibody is 40 mg, 0.8 mL of the composition having the antibody concentration of 50 mg/ml, or 0.4 mL of the composition having the antibody concentration of 100 mg/ml). In another embodiment, the pharmaceutical formulation may be prepared in common bulk formulations. The concentrations of the components of the pharmaceutical composition may be adjusted to a level higher than that required for administration, and properly diluted prior to administration.

ADVANTAGEOUS EFFECTS OF DISCLOSURE

The present disclosure provides an aqueous liquid composition, specifically, a pharmaceutical composition capable of stably maintaining physical, chemical, and/or biological efficacy of a protein, e.g., an anti-TNFa antibody. The composition may prevent formation of polymers and/or aggregates, formation of degradation products (fragments), denaturation to charge variants, oxidation of amino acid residues, which may occur during the preservation of proteins, thereby stably maintaining the pharmaceutical efficacy of the protein.

BRIEF DESCRIPTION OF DRAWINGS

FIG. 1 is a graph showing oxidation rates (average value: n=3) of an amino acid ($Met_{256}$) in a protein (adalimumab: 100 mg/mL) formulation according to one embodiment under photostress conditions, according to the type and concentration of amino acid;

FIG. 2A is a graph showing %HMW of a protein in an antibody formulation according to one embodiment under photostress conditions, according to the type and concentration of amino acid;

FIG. 2B is a graph showing %Monomer of a protein in an antibody formulation according to one embodiment under photostress conditions, according to the type and concentration of amino acid;

FIG. 2C is a graph showing %LMW of a protein in an antibody formulation according to one embodiment under photostress conditions, according to the type and concentration of amino acid;

FIG. 3 is a graph showing oxidation rates (average value: n=2) of an amino acid ($Met_{256}$) in a protein (adalimumab: 50 mg/mL) formulation according to one embodiment under photostress conditions, according to the presence or absence of histidine;

FIG. 4 is a graph showing high molecular weight% (%HMW) (average value: n=3) of a protein in a protein (adalimumab: 50 mg/mL) formulation according to one embodiment during preservation at 40±2 °C for 2 weeks, according to the type and concentration of surfactant, as measured by SE-HPLC analysis;

FIG. 5 is a graph showing %HMW (average value: n=3) of a protein in a protein (adalimumab: 50 mg/mL) formulation according to one embodiment under freeze-thaw conditions (5 cycles), according to the type and concentration of surfactant, as measured by SE-HPLC analysis;

FIG. 6 is a graph showing %HMW (average value: n=3) of a protein in a protein (adalimumab: 50 mg/mL) formulation according to one embodiment during preservation at 40±2 °C for 4 weeks, according to the presence or absence of histidine and the type of surfactant, as measured by SE-HPLC analysis;

FIG. 7 is a graph showing %HMW (average value: n=3) of a protein in a protein (adalimumab: 100 mg/mL) formulation according to one embodiment during preservation at 40±2 °C for 4 weeks, according to the presence or absence of histidine and the type of surfactant, as measured by SE-HPLC analysis;

FIG. 8 is a graph showing %Acidic (a content ratio of acidic variants) (average value: n=3) of a protein in a protein (adalimumab: 50 mg/mL) formulation according to one embodiment during preservation at 40±2 °C for 4 weeks, according to the presence or absence of histidine and the type of surfactant;

FIG. 9 is a graph showing %Acidic (average value: n=3) of a protein in a protein (adalimumab: 100 mg/mL) formulation according to one embodiment during preservation at 40±2 °C for 4 weeks, according to the presence or absence of histidine and the type of surfactant;

FIG. 10 is a graph showing %Acidic (average value: n=3) of a protein in a protein (adalimumab: 50 mg/mL) formulation according to one embodiment under freeze-thaw conditions (5 cycles), according to the type and concentration of surfactant, as measured by icIEF analysis;

FIGS. 11A to 11D show quality test results (11A: %HMW, 11B: %Total purity, 11C: TNFα binding activity, and 11D: TNFα neutralizing activity) of a prefilled syringe pre-filled with a protein (adalimumab; 50 mg/mL) formulation according to one embodiment, as tested by temperature excursions; and

FIGS. 12A to 12E show quality test results (12A: %HMW, 12B: %Total purity, 12C: %Main, 12D: TNFα binding activity, and 12E: TNFα neutralizing activity) of a prefilled syringe pre-filled with a protein (adalimumab; 50 mg/mL) formulation according to one embodiment, as tested by storage stability at room temperature.

BEST MODE

Hereinafter, the present disclosure will be described in more detail with reference to Examples and Experimental Examples. However, these Examples and Experimental Examples are only for illustrating the present disclosure, and they are not to be construed as limiting the present disclosure.

REFERENCE EXAMPLES

Reference Example 1. SE-HPLC Analysis

Size exclusion-high performance liquid chromatography (SE-HPLC) analysis was performed using the HPLC system of Waters Corporation according to the manufacturer's manual. A total of three peaks were separated depending on retention times (molecular weights of proteins). These three peaks accounted for a HMW peak (protein aggregation), a monomer peak, and an LMW peak (protein degradation), in order of shorter retention times (larger molecular weights of proteins).

- %HMW (High molecular weight%) = {area of HMW/area of (HMW + monomer + LMW)}*100)

- %Monomer (Monomer weight%) = {area of monomer/area of (HMW + monomer + LMW)}*100)

- %LMW(Low molecular weight%) = {area of LMW/area of (HMW + monomer + LMW)}*100)

Reference Example 2. Photostress

Formulations were subjected to a sample stability test under photostress conditions of Table 1 below.

[Table 1]

| Base group (Base, initial) | Dark control * | Light-exposed group (Light-exposed) |
|---|---|---|
| N/A (Not applicable) | Cool White Fluorescent Lamp : ≥1.2 M lux hours | Cool White Fluorescent Lamp : ≥1.2 M lux hours |
| N/A (Not applicable) | Near Ultraviolet Lamp : ≥ 200 Watt hours/square meter | Near Ultraviolet Lamp : ≥ 200 Watt hours/square meter |

(continued)

| Base group (Base, initial) | Dark control * | Light-exposed group (Light-exposed) |
|---|---|---|
| Chamber Environmental Conditions : 5±3 °C | Chamber Environmental Conditions: 25±2 °C/60±5% RH(relative humidity) | Chamber Environmental Conditions: 25±2 °C/60±5% RH(relative humidity) |
| * For a dark control, the sample was wrapped with foil, and then the experiment was performed under the above conditions.<br>- The International Council for Harmonisation of Technical Requirements for Pharmaceuticals for Human Use (ICH) guideline, 'Q1B Stability Testing: Photostability Testing of New Drug Substances and Products' | | |

Reference Example 3. Measurement of %Oxidation Rate (%Oxidation Level) of Amino Acid Residue

With regard to %oxidation rate of amino acid residues in the protein included in the formulation, the mass of the 256th methionine amino acid residue of adalimumab heavy chain was measured using liquid-chromatography-mass spectrometry (LC-MS) of Waters Corporation. After performing the LC-MS experiment, a relative %oxidation rate was analyzed using MassLynx™ program.

$$\text{Relative \%Oxidation} = \text{(Control intensity of oxidized peptide*100)/\{(Control intensity of non-oxidized peptide)+(Control intensity of oxidized peptide)}$$

Reference Example 4. CEX-HPLC Analysis

Cation Exchange-High Performance Liquid Chromatography (CEX-HPLC) analysis was performed using the HPLC system of Waters Corporation according to the manufacturer's manual. A total of three peaks were separated depending on retention times (charges of proteins). These three peaks accounted for an acidic peak, a main peak, and a basic peak, in order of shorter retention times.

$$- \text{Acidic\%(content ratio of Acidic variants, \%Acidic)} = \{\text{area of acidic/area of (acidic + main + basic)}\}*100)$$

$$- \text{Main\%(content ratio of Main protein, \%Main)} = \{\text{area of main/area of (acidic + main + basic)}\} *100)$$

$$- \text{Basic\%(content ratio of Basic variants, \%Basic)} = \{\text{area of basic/area of (acidic + main + basic)}\}*100)$$

Reference Example 5. icIEF Analysis

Imaged capillary isoelectric focusing (icIEF) analysis method identifies charge variants of a protein according to their isoelectric point (pI) in a capillary containing ampholytes, and the analysis was performed by analyzing an acidic isoform, a main isoform, and a basic isoform using the icIEF instrument (ProteinSimple, iCE3) according to the manufacturer's manual.

Reference Example 6. Quality (Stability) Test of Antibody-Containing Product (Antibody-Prefilled Syringe)

Stability of antibody-containing product samples was evaluated by methods summarized in Table 2 below:

[Table 2]

| Category | Formulation stability evaluation items | Method | Description |
|---|---|---|---|
| Visible features | Color | Visual inspection | Color evaluation of sample using Brown standards B1-B8 |
| | Clarity | Turbidity measurement (HACH Model 2100AN) | Clarity evaluation of sample |
| | Visible particles | Visual inspection | Measurement of number of visible particles in sample |
| pH | pH | pH measurement (Thermo Scientific Orion star A211) | pH evaluation of sample |
| Chemical analysis | Protein concentration | $A_{280}$ measurement (UV-VIS spectrophotometer) | Concentration evaluation of protein (antibody) in sample |
| Physicochemical analysis | HMW impurity | SE-HPLC | Measurement of content ratio of high-molecular-weight (HMW) impurity in sample |
| | Total purity | CE-SDS | Measurement of total impurity in sample |
| | LMW impurity | CE-SDS | Measurement of content ratio of low-molecular-weight (LMW) impurity (e.g., NGHC) in sample |
| | Charge isoforms | CEX-HPLC, iclEF | Measurement of charge variants including acidic, main, basic variants |
| Subvisible particles | Subvisible particles | HIAC 9703 + HRLD-400 | Measurement of number of particles having an average particle diameter of 25 $\mu$m or more, 10 $\mu$m or more, 8 $\mu$m or more, 5 $\mu$m or more, 2 $\mu$m or more |
| Biological activity | TNF-alpha binding activity | FRET | Measurement of relative binding affinity to Reference standard |
| | TNF-alpha neutralizing activity | Luciferase reporter gene system assay | Measurement of relative potency to Reference standard |

6.1. Presence of visible particles, Color, and clarity

Through visual inspection, the presence of visible particles in the formulation and color (Brown standard: B1-B8) were evaluated. Clarity of the formulation was measured using a turbidimeter (HACH Model 2100AN).

6.2. pH

pH of the sample was measured using a Thermo Scientific Orion star A211 (a combination of pH electrode and temperature probe; Thermo Scientific) adjusted to pH 4, 7, and 10 before use.

6.3. Protein concentration

Protein concentrations were measured by absorbance at a wavelength of 280 nm. In detail, all tested samples were diluted to a concentration of 1 mg/mL, based on the expected concentration of each sample in a 0.9%(w/v) NaCl solution, and absorbance at 280 nm (A280) was determined by a UV-VIS spectrophotometer (Agilent), and the obtained results were normalized using a 0.9%(w/v) NaCl solution as a blank solution.

6.4. High-molecular-weight (HMW) impurity

High-molecular-weight impurity in the sample was measured using size exclusion-high performance liquid chromatography (SE-HPLC) (Reference Example 1).

6.5. Total purity

Total purity of the sample was evaluated by measuring the prevalence of non-glycosylated heavy chains (NGHC) using capillary electrophoresis-sodium dodecyl sulfate (CE-SDS) under non-reducing conditions.

6.6. Charge variants

Levels of charge variants in the sample were measured using CEX-HPLC (Reference Example 4) and iclEF (Reference Example 5).

6.7. TNF-$\alpha$ binding activity (TNF-$\alpha$ binding affinity)

TNF-$\alpha$ binding activity of the antibody (adalimumab) included in the sample was measured by a competitive inhibition binding assay (CIBA) using fluorescence resonance energy transfer (FRET). TNF-$\alpha$ binding was measured by time-resolved fluorescence resonance energy transfer (TR-FRET). Adalimumab was labeled with a fluorescent europium (Eu) chelate (PerkinElmer), and human TNF-$\alpha$ was labeled with a Cy5 fluorophore, which were used in analysis. Eu-labeled adalimumab bound to Cy5-labeled TNF-$\alpha$ generates a fluorescent signal. When an antibody (adalimumab) in a fluorescent unlabeled sample is added thereto, the unlabeled antibody competes with Eu-labeled adalimumab for binding to Cy5-labeled TNF-$\alpha$. Therefore, as the antibody in the sample binds to Cy5-labeled TNF-$\alpha$, fluorescence generation is inhibited, and the measured fluorescence signal level is inversely proportional to the binding level of the unlabeled antibody in the sample to TNF-$\alpha$. Eu-labeled adalimumab and Cy5-labeled TNF-$\alpha$ at a predetermined concentration and volume were sequentially added to an assay plate containing a reference standard and samples, followed by incubation at room temperature with gentle agitation, and the levels of generated fluorescence signal were measured by time-resolved fluorimetry (EnVision™, PerkinElmer). The TNF-$\alpha$ binding activity of adalimumab in the sample was calculated as a relative value (%relative binding activity) to the reference standard using a Parallel Line Analysis (PLA) software (Stegmann Systems). The reference standard means a reference substance having a reference value to which 100% is assigned, and the dose curves of the sample and the reference standard may be compared and analyzed through the PLA program to obtain %relative binding activity.

6.8. TNF-$\alpha$ neutralizing activity (TNF-$\alpha$ neutralization)

The TNF-$\alpha$ neutralizing activity of the antibody (adalimumab) included in the sample was measured using a luciferase reporter gene system. The sample was preincubated with TNF-$\alpha$, and then incubated together with a cell line engineered to include a luciferase reporter gene (293-NF-$\kappa$B-luc cell line (CVCL_KS34) engineered to include a human NF-$\kappa$B response element upstream of the luciferase reporter gene, in which TNF-$\alpha$ binds to TNFR on the cell surface, thereby activating NF-$\kappa$B signaling and promoting luciferase reporter gene expression). The sample was mixed with human TNF-$\alpha$ at a ratio of 1:1 (v/v) in a 96-well tissue culture plate, and incubated at room temperature for 30 minutes to 90 minutes. Thereafter, the prepared cells were put into the 96-well plate and incubated for 24 hours, and then the luminescence signal was measured using a Steady-Glo® Luciferase Assay System (Promega) on a microplate reader (EnVision™, PerkinElmer), thereby measuring TNF-$\alpha$ neutralization potency of adalimumab. The TNF-$\alpha$ neutralizing activity of adalimumab was calculated as a relative value (%relative potency) to a reference standard using a Parallel Line Analysis (PLA) software (Stegmann Systems). The reference standard means a reference substance having a reference value to which 100% is assigned.

6.9. Number of particles per size

Particles with an average particle diameter of 2 $\mu$m or more, 5 $\mu$m or more, 8 $\mu$m or more, 10 $\mu$m or more, and 25 $\mu$m or more were counted using HIAC 9703+ and HRLD 400 instrument (Beckman Coulter, Inc.).

6.10. Endotoxin

Based on the reaction in which endotoxin activates limulus amebocyte lysate (LAL) to cause gelation, endotoxin was detected and quantified using a Bio Tek Elx808 IU Microplate Reader System (Bio Tek Instruments).

Example 1. Amino acid screening

1-1. Photostability of Formulation Containing High Concentration of Protein Under Photostress Conditions

By evaluating stability of a protein formulation according to the type and concentration of amino acid, the type and concentration range of amino acid showing advantageous effects on the stability of the protein formulation were identified. In detail, photostability of the formulation containing a high concentration of protein under photostress conditions was examined in terms of antioxidant and anti-aggregation effects.

1-1-1. Preparation of formulation containing high concentration of protein

Aqueous liquid formulations including 100 mg/mL of an anti-TNFa antibody (Adalimumab; anti-TNFa monoclonal antibody; CAS Registry Number: 331731-18-1) as a protein and having the compositions of the following Table 3 were prepared:

[Table 3]

| Sample No. | Concentration of protein (mg/mL) | pH | Buffer | Excipient 1 | Excipient 2 | Surfactant |
|---|---|---|---|---|---|---|
| 1 | 100 | 5.2 | 2.6 mM Na-phosphate | 59 mM His | 3.0% mannitol | 0.08% PS 20 |
| 2 | | | 2.6 mM Na-phosphate + 5 mM Na-succinate | | | |
| 3 | | | | N/A | 5.0% mannitol | |
| 4 | | | | 59 mM Arg-HCl | 3.0% mannitol | |
| 5 | | | | 10 mM His | 4.5% mannitol | |
| 6 | | | | 80 mM His | 2.0% mannitol | |
| 7* | | | N/A | N/A | 4.2% mannitol | 0.1% PS80 |

[00267] (*: a sample including the same formulation ingredients as Humira® including 100 mg/mL of adalimumab;
[00268] PS 20: polysorbate 20, PS 80: polysorbate 80;
[00269] N/A: not applicable;
[00270] %: %(w/v))

1-1-2. Anti-oxidation effect

With respect to each of the formulations prepared according to Table 3, an oxidation rate ($\%Met_{256}$) of Met256 (methionine residue at position 256) in the protein under photostress conditions was measured. The photostress conditions were prepared with reference to Reference Example 2, and the oxidation rate ($\%Met_{256}$) of Met256 in the protein was measured with reference to Reference Example 3.

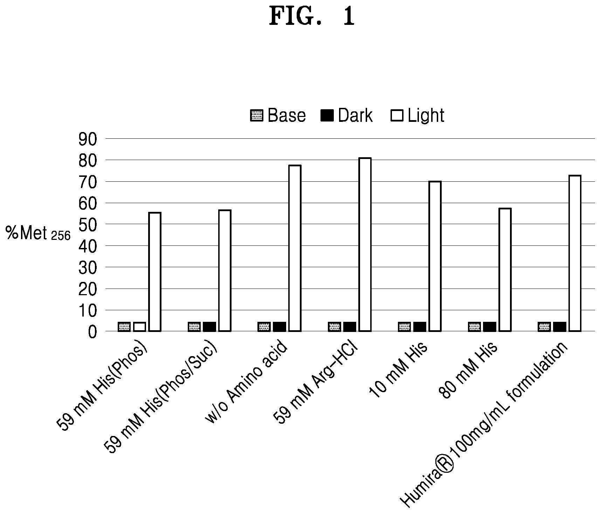

The oxidation rate measurement was repeated three times (n=3), and the average values of the obtained results are shown in the following Table 4, and FIG. 1:

[Table 4]

| Oxidation rate (%$Met_{256}$) of amino acid ($Met_{256}$) and Change ($\Delta$%$Met_{256}$) in amino acid oxidation rate under photostress conditions | | | | | |
|---|---|---|---|---|---|
| Sample No. | %$Met_{256}$ | | | $\Delta$%$Met_{256}$ | |
| | Base | Dark-control | Light-exposed | [Light-exposed %$Met_{256}$] - [Base %$Met_{256}$] | [Light-exposed %$Met_{256}$] - [Dark-control %$Met_{256}$] |
| 1 | 4.23 | 4.43 | 55.80 | 51.57 | 51.37 |
| SD | 0.06 | 0.31 | 1.04 | 1.08 | 1.34 |
| 2 | 4.67 | 4.57 | 57.07 | 52.40 | 52.50 |
| SD | 0.42 | 0.32 | 0.49 | 0.10 | 0.66 |
| 3 | 4.37 | 4.57 | 77.77 | 73.40 | 73.20 |
| SD | 0.12 | 0.40 | 2.99 | 3.04 | 3.35 |
| 4 | 4.23 | 4.57 | 80.83 | 76.60 | 76.27 |
| SD | 0.21 | 0.32 | 4.03 | 3.82 | 3.72 |
| 5 | 4.07 | 4.57 | 70.23 | 66.17 | 65.67 |
| SD | 0.21 | 0.25 | 0.35 | 0.38 | 0.31 |
| 6 | 4.20 | 4.80 | 57.63 | 53.43 | 52.83 |
| SD | 0.26 | 0.46 | 3.51 | 3.62 | 3.88 |
| 7 | 4.13 | 4.43 | 73.27 | 69.13 | 68.83 |
| SD | 0.21 | 0.35 | 2.11 | 1.92 | 1.76 |

As confirmed from the above results, the $Met_{256}$ oxidation rate and a change thereof according to the type of amino acid under the light-exposed conditions were observed in this order of Arg-HCl > Amino acid-free > His, indicating that the histidine-containing formulations are more suitable than the amino acid-free formulations or the Arg-HCl-containing formulations, in terms of photostability of formulation. In addition, the histidine-containing formulations exhibited remarkably low amino acid ($Met_{256}$) oxidation rates and changes thereof to have excellent antioxidant effect, as compared with the amino acid-free formulation (Sample No. 7) having the same composition as the commercially available Humira® 100 mg/mL (pH 5.2, 4.2% mannitol, 0.1% PS 80).

In addition, as a result of analysis according to the histidine concentration, the change in the amino acid oxidation rate was observed in the order of 10 mM His > 59 mM His ≒ 80 mM His, indicating that formulations containing 59 mM or more of His had higher antioxidant effects.

As above, it was confirmed that histidine has activity as an anti-oxidant agent. In particular, when the histidine content in the antibody formulation is 10 mM or more, or more than 10 mM, and 80 mM or less (e.g., about 59 mM), the highest antioxidant effect was observed.

1-1-3. Anti-aggregation effect

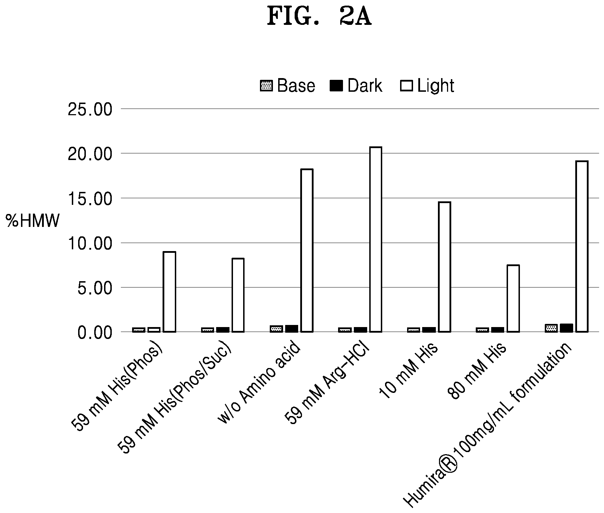

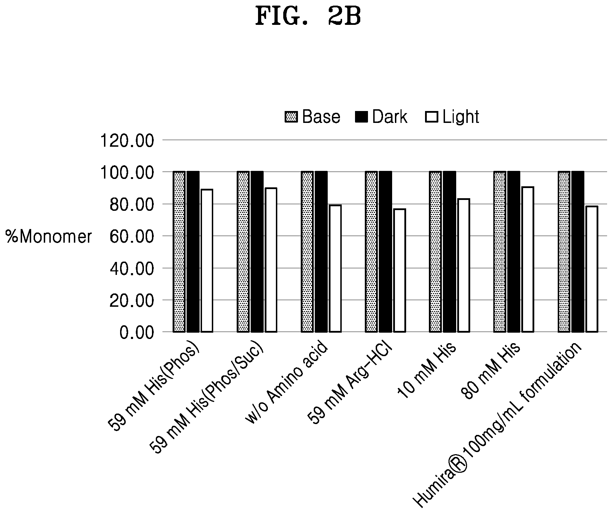

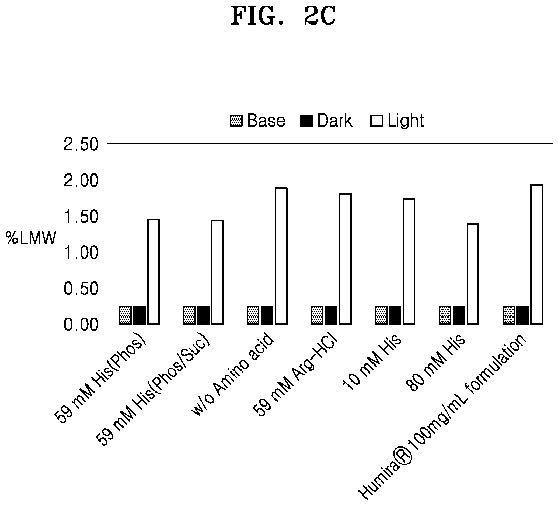

With respect to the formulations prepared according to Table 3, high molecular weight% (%HMW), monomer weight% (%monomer), and low molecular weight% (%LMW) of the protein under photostress conditions of Reference Example 2 were measured by SE-HPLC analysis according to Reference Example 1. The test was repeated three times (n=3), and the average values of the obtained results are shown in the following Tables 5 to 7, and FIGS. 2A to 2C:

[Table 5]

| | %HMW under photostress conditions | | | | |
|---|---|---|---|---|---|
| Sample No. | %HMW | | | Δ%HMW | |
| | Base | Dark-control | Light-exposed | [Light-exposed %HMW]-[Base %HMW] | [Light-exposed %HMW] - [Dark-control %HMW] |
| 1 | 0.35 | 0.46 | 8.92 | 8.57 | 8.46 |
| SD | 0.01 | 0.02 | 0.15 | 0.14 | 0.13 |
| 2 | 0.33 | 0.43 | 8.19 | 7.86 | 7.76 |
| SD | 0.00 | 0.01 | 0.12 | 0.12 | 0.12 |
| 3 | 0.65 | 0.79 | 18.13 | 17.49 | 17.34 |
| SD | 0.02 | 0.03 | 0.61 | 0.60 | 0.58 |
| 4 | 0.40 | 0.50 | 20.79 | 20.39 | 20.29 |
| SD | 0.00 | 0.01 | 0.85 | 0.85 | 0.86 |
| 5 | 0.47 | 0.58 | 14.53 | 14.07 | 13.96 |
| SD | 0.01 | 0.01 | 0.34 | 0.34 | 0.34 |
| 6 | 0.31 | 0.40 | 7.45 | 7.14 | 7.05 |
| SD | 0.00 | 0.01 | 0.20 | 0.20 | 0.20 |
| 7 | 0.77 | 0.95 | 19.14 | 18.38 | 18.20 |
| SD | 0.01 | 0.01 | 0.26 | 0.26 | 0.26 |

[Table 6]

| | %Monomer under photostress conditions | | | | |
|---|---|---|---|---|---|
| Sample No. | %Monomer | | | Δ%Monomer | |
| | Base | Dark-control | Light-exposured | [Light-exposured %Monomer] - [Base %Monomer] | [Light-exposured %Monomer] - [Dark-control %Monomer] |
| 1 | 99.40 | 99.29 | 89.62 | -9.77 | -9.66 |
| SD | 0.01 | 0.02 | 0.17 | 0.16 | 0.15 |
| 2 | 99.42 | 99.31 | 90.37 | -9.05 | -8.94 |
| SD | 0.00 | 0.01 | 0.11 | 0.11 | 0.12 |
| 3 | 99.10 | 98.95 | 79.97 | -19.13 | -18.98 |
| SD | 0.01 | 0.03 | 0.62 | 0.61 | 0.60 |
| 4 | 99.35 | 99.25 | 77.40 | -21.95 | -21.85 |
| SD | 0.00 | 0.01 | 0.83 | 0.83 | 0.84 |
| 5 | 99.28 | 99.17 | 83.73 | -15.56 | -15.44 |
| SD | 0.01 | 0.02 | 0.35 | 0.34 | 0.35 |
| 6 | 99.44 | 99.40 | 91.15 | -8.29 | -8.25 |
| SD | 0.02 | 0.11 | 0.23 | 0.25 | 0.30 |
| 7 | 98.98 | 98.80 | 78.93 | -20.05 | -19.88 |
| SD | 0.01 | 0.01 | 0.24 | 0.24 | 0.24 |

[Table 7]

| Sample No. | %LMW | | | Δ%LMW | |
|---|---|---|---|---|---|
| | Base | Dark-control | Light-exposed | [Light-exposed %LMW] - [Base %LMW] | [Light-exposed %LMW] - [Dark-control %LMW] |
| 1 | 0.25 | 0.25 | 1.46 | 1.21 | 1.21 |
| SD | 0.00 | 0.00 | 0.02 | 0.02 | 0.02 |
| 2 | 0.25 | 0.25 | 1.44 | 1.19 | 1.19 |
| SD | 0.00 | 0.01 | 0.02 | 0.02 | 0.02 |
| 3 | 0.25 | 0.26 | 1.89 | 1.64 | 1.64 |
| SD | 0.01 | 0.01 | 0.02 | 0.03 | 0.03 |
| 4 | 0.25 | 0.26 | 1.81 | 1.56 | 1.55 |
| SD | 0.00 | 0.01 | 0.02 | 0.02 | 0.02 |
| 5 | 0.25 | 0.26 | 1.74 | 1.49 | 1.48 |
| SD | 0.00 | 0.01 | 0.02 | 0.02 | 0.02 |
| 6 | 0.25 | 0.20 | 1.40 | 1.15 | 1.20 |
| SD | 0.01 | 0.10 | 0.03 | 0.04 | 0.11 |
| 7 | 0.25 | 0.25 | 1.93 | 1.68 | 1.68 |
| SD | 0.01 | 0.00 | 0.03 | 0.03 | 0.03 |

*%LMW under photostress conditions*

As confirmed from the above results, %HMW and change of %HMW under the light-exposed conditions were observed in this order of Arg-HCl > Amino acid-free > His, indicating that the histidine-containing formulations exhibited more improved anti-aggregation effect than the amino acid-free formulations or the Arg-HCl-containing formulations, and this effect shows a similar trend when compared to Sample 7 having the same composition as Humira® 100 mg/mL (pH 5.2, 4.2% mannitol, 0.1% PS 80), which is an amino acid-free formulation.

In addition, %HMW and change of %HMW under the light-exposed conditions according to His concentrations were observed in the order of 10 mM His > 59 mM His ≒ 80 mM His, indicating that formulations containing 59 mM to 80 mM of His had higher anti-aggregation effects than the formulation containing 10 mM of His.

This indicates that when the His concentration is 59 mM or more, more excellent anti-aggregation effects are obtained. In addition, %Monomer and %LMW results also showed that histidine-containing formulations (Sample Nos. 1, 2, 5, and 6) had higher anti-aggregation effects than amino acid-free formulations or formulations containing amino acids (e.g., arginine) other than histidine.

1-2. Photostability of Formulation Containing Low Concentration of Protein Under Photostress Conditions

Photostability of the formulations containing histidine, which was confirmed in Example 1-1 to have excellent photostability, and low concentration of protein were evaluated under photostress conditions.

Aqueous liquid formulations including 50 mg/mL of an anti-TNFa antibody (Adalimumab; anti-TNFa monoclonal antibody; CAS Registry Number: 331731-18-1) as a protein and having the compositions of the following Table 8 were prepared:

[Table 8]

| Sample No. | Concentration of protein (mg/mL) | pH | Buffer | Excipient 1 | Excipient 2 | Surfactant |
|---|---|---|---|---|---|---|
| 1 | 50 | 5.2 | 10 mM Na- citrate | 59 mM His | 2.5% sorbitol | 0.08% PS 20 |
| 2 | | | | N/A | | |

(N/A: not applicable; %: %(w/v))

With respect to the prepared histidine (His)-containing formulations and histidine-free formulations, an oxidation rate ($\%Met_{256}$) of $Met_{256}$ (methionine residue at position 256), which is known as a main oxidized site in the protein, under photostress conditions was measured. The photostress conditions were prepared with reference to Reference Example 2, and the oxidation rate ($\%Met_{256}$) of $Met_{256}$ in the protein was measured with reference to Reference Example 3.

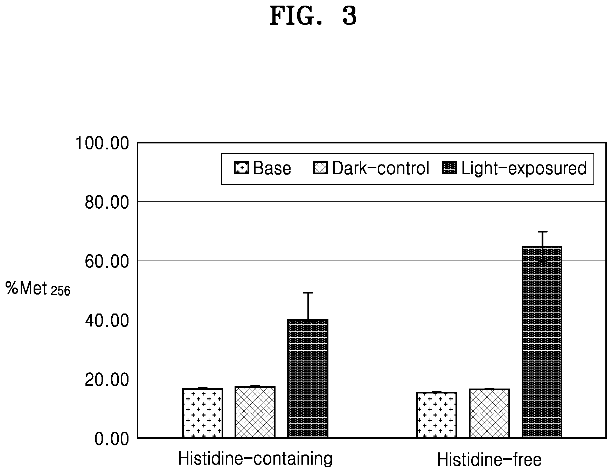

The oxidation rate measurement was repeated twice (n=2), and the average values of the obtained results are shown in the following Table 9, and FIG. 3:

[Table 9]

| Oxidation rate ($\%Met_{256}$) of amino acid ($Met_{256}$) and Change ($\Delta\%Met_{256}$) in amino acid oxidation rate under photostress conditions | | | | | |
|---|---|---|---|---|---|
| Sample No. | | $\%Met_{256}$ | | | $\Delta\%Met_{256}$ | |
| Sample No. | | Base | Dark-control | Light-exposed | [Light-exposed $\%Met_{256}$] - [Base $\%Met_{256}$] | [Light-exposed $\%Met_{256}$] - [Dark-control $\%Met_{256}$] |
| 1 | Average | 16.50 | 17.10 | 40.40 | 23.90 | 23.30 |
| 1 | SD (N=2) | 0.14 | 0.42 | 0.57 | 0.42 | 0.99 |
| 2 | Average | 15.10 | 16.35 | 64.90 | 49.80 | 48.55 |
| 2 | SD (N=2) | 0.28 | 0.07 | 4.95 | 4.67 | 4.88 |

As shown in Table 9, it was confirmed that $\%Met_{256}$ of the histidine-containing formulation even with a low protein concentration under light-exposed conditions was 24.50% lower than that of the histidine-free formulation. In addition, as a result of analyzing the change ($\Delta\%Met_{256}$) in the amino acid oxidation rate of the light-exposed group, as compared with a base group (Base) and a dark control (Dark control), according to the presence or absence of histidine, $\Delta\%Met_{256}$ of the histidine-containing formulation were 25.90% ($\Delta\%Met_{256}$ relative to the base group) and 25.25% ($\Delta\%Met_{256}$ relative to dark control) lower than that of the histidine-free formulation. These results showed a similar tendency to the experimental results of the formulations containing a high concentration of protein of Example 1-1. Consequently, it was confirmed that the histidine-containing formulations had the excellent antioxidant effect on amino acid residues in the protein, as compared with the histidine-free formulation, demonstrating the function of histidine as an anti-oxidant agent in the protein formulations.

Example 2. Surfactant Screening

2-1. Stability of protein formulation according to type of surfactant

To evaluate stability of the protein formulation according to the type of surfactant, aqueous liquid formulations including 50 mg/mL of an anti-TNFa antibody (Adalimumab; anti-TNFa monoclonal antibody; CAS Registry Number: 331731-18-1) as a protein and having the compositions of the following Table 10 were prepared:

[Table 10]

| Sample No. | Concentration of protein (mg/mL) | pH | Buffer | Excipient 1 | Excipient 2 | Surfactant |
|---|---|---|---|---|---|---|

(continued)

| Sample No. | Concentration of protein (mg/mL) | pH | Buffer | Excipient 1 | Excipient 2 | Surfactant |
|---|---|---|---|---|---|---|
| 1 | 50 | 5.2 | 10 mM Na-citrate | 59 mM His | 2.5% sorbitol | 0.04% PS 20 |
| 2 | | | | | | 0.08% PS 20 |
| 3 | | | | | | 0.1% PS 20 |
| 4 | | | | | | 0.2% PS 20 |
| 5 | | | | | | 0.04% PS 80 |
| 6 | | | | | | 0.08% PS 80 |
| 7 | | | | | | 0.1% PS 80 |
| 8 | | | | | | 0.2% PS 80 |
| [00307] (PS 20: polysorbate 20, PS 80: polysorbate 80; [00308] %: %(w/v)) | | | | | | |

2-1-1. Stability of formulation under heat stress conditions

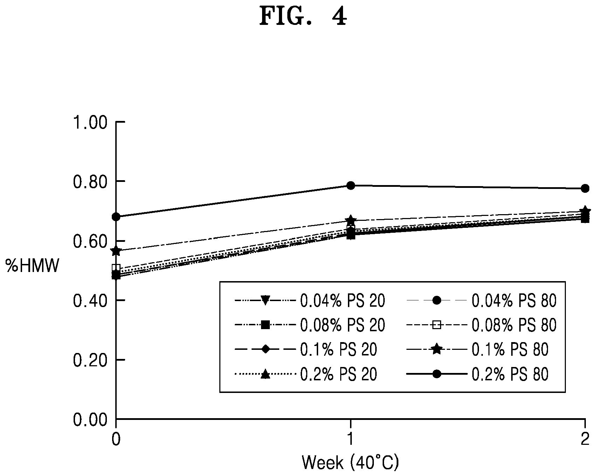

The formulations prepared in Example 2-1 were stored at 40±2 °C for 2 weeks, and then high molecular weight% (%HMW) of the protein in the formulation at 0th week (initial), 1st week, and 2nd weeks was measured by SE-HPLC analysis according to Reference Example 1. The measurement was repeated three times (n=3), and the average values of the obtained results are shown in the following Table 11, and FIG. 4:

[Table 11]

| %HMW upon Storage over Two Weeks at 40 °C | | | | |
|---|---|---|---|---|
| Sample No. | | %HMW | | |
| | | Initial | 1 Wk | 2 Wk |
| 1 | Average | 0.48 | 0.62 | 0.67 |
| | SD (N=3) | 0.00 | 0.01 | 0.00 |
| 2 | Average | 0.49 | 0.62 | 0.67 |
| | SD (N=3) | 0.00 | 0.00 | 0.00 |
| 3 | Average | 0.49 | 0.63 | 0.68 |
| | SD (N=3) | 0.00 | 0.01 | 0.00 |
| 4 | Average | 0.51 | 0.64 | 0.69 |
| | SD (N=3) | 0.01 | 0.01 | 0.00 |
| 5 | Average | 0.49 | 0.62 | 0.67 |
| | SD (N=3) | 0.00 | 0.00 | 0.01 |
| 6 | Average | 0.51 | 0.63 | 0.68 |
| | SD (N=3) | 0.01 | 0.01 | 0.01 |
| 7 | Average | 0.57 | 0.67 | 0.70 |
| | SD (N=3) | 0.01 | 0.01 | 0.01 |
| 8 | Average | 0.68 | 0.79 | 0.78 |
| | SD (N=3) | 0.01 | 0.01 | 0.02 |

As shown in Table 11 and FIG. 4, initial %HMW of the formulations including polysorbate 20 (Samples 1~4) was maintained at 0.48% to 0.51% with increasing concentrations, whereas initial %HMW of the formulations including polysorbate 80 (Samples 5~8) increased from 0.49% to 0.68% with increasing concentrations. In particular, initial %HMW of the formulation including 0.2% polysorbate 20 (Sample 4) was 0.17% lower than that of the formulation including 0.2% polysorbate 80 (Sample 8), and %HMW at 2nd weeks of the formulation including 0.2% polysorbate 20 was 0.09% lower than that of the formulation including 0.2% polysorbate 80.

2-1-2. Stability of formulation under freeze-thaw conditions

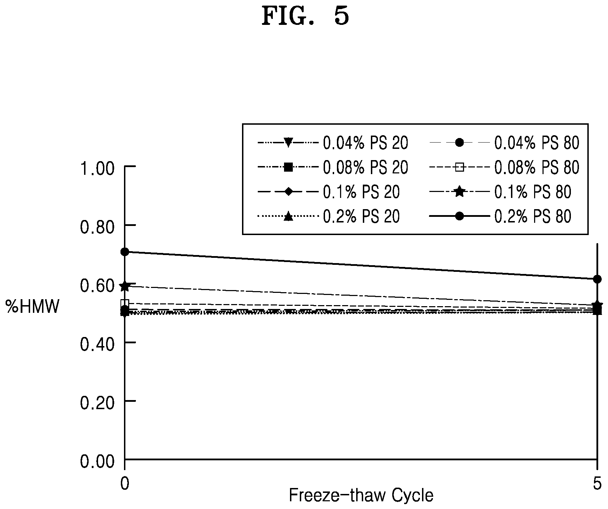

High molecular weight% (%HMW) of proteins in the formulations prepared in Example 2-1 were measured under freeze-thaw conditions (Freeze/thaw, 5 cycles; FT5; each cycle: 'frozen at -70 °C±10 °C for 18 hours or longer' + 'thawed at room temperature (25 °C) for 1 hour or longer') by SE-HPLC analysis according to Reference Example 1. The experiment was repeated three times (n=3) and the average values of the results are shown in the following Table 12 and FIG. 5.

[Table 12]

| %HMW under FT5 (Freeze/thaw, 5 cycle) conditions | | | |
|---|---|---|---|
| Sample No. | | %HMW | |
| | | Initial | FT5 |
| 1 | Average | 0.48 | 0.48 |
| | SD (N=3) | 0.00 | 0.00 |
| 2 | Average | 0.49 | 0.48 |
| | SD (N=3) | 0.00 | 0.00 |
| 3 | Average | 0.49 | 0.48 |
| | SD (N=3) | 0.00 | 0.01 |
| 4 | Average | 0.51 | 0.49 |
| | SD (N=3) | 0.01 | 0.00 |
| 5 | Average | 0.49 | 0.49 |
| | SD (N=3) | 0.00 | 0.01 |
| 6 | Average | 0.51 | 0.49 |
| | SD (N=3) | 0.01 | 0.00 |
| 7 | Average | 0.57 | 0.50 |
| | SD (N=3) | 0.01 | 0.01 |
| 8 | Average | 0.68 | 0.59 |
| | SD (N=3) | 0.01 | 0.02 |

As shown in Table 12 and FIG. 5, initial %HMW of the formulations including polysorbate 20 (Samples 1~4) was maintained at 0.48% to 0.51% with increasing concentrations, whereas initial %HMW of the formulations including polysorbate 80 (Samples 5~8) increased from 0.49% to 0.68% with increasing concentrations. In particular, initial %HMW and %HMW after F5 of the formulation including 0.2% polysorbate 20 (Sample 4) was 0.17% and 0.1% lower than that of the formulation including 0.2% polysorbate 80 (Sample 8), respectively, indicating excellent stability.

2-2. Evaluation of stability of formulations containing histidine and polysorbate 20

2-2-1. Preparation of formulation

With respect to the protein formulations including histidine and polysorbate 20, which were confirmed in Examples 1 and 2-1 to have excellent formulation stability, various exemplary formulations were prepared, and thermal stability thereof was evaluated.

To this end, experimental formulations and control formulations were prepared according to the following compositions:

- Experimental Formulation 1: 50 mg/ml of adalimumab, pH 5.2, 10 mM Na-citrate, 59 mM histidine, 2.5%(w/v) sorbitol, 0.08%(w/v) polysorbate 20;
- Experimental Formulation 2: 100 mg/ml of adalimumab, pH 5.2, 2.5 mM Na-citrate, 59 mM histidine, 2.5%(w/v) sorbitol, 0.08%(w/v) polysorbate 20;
- Control Formulation 1 (a formulation having the same composition as Cyltezo®): 50 mg/ml of adalimumab, pH 5.2, 24.7 mM Na-acetate, 8.1% trehalose, 0.1% polysorbate 80;
- Control Formulation 2 (a formulation having the same composition as Amgevita®): 50 mg/ml of adalimumab, pH 5.2, 10 mM acetate, 9.0% sucrose, 0.1% polysorbate 80
- Control Formulation 3 (a formulation having the same composition as Cyltezo®, except that 100 mg/ml of adalimumab was included instead of 50 mg/ml of adalimumab): 100 mg/ml of adalimumab, pH 5.2, 24.7 mM Na-acetate, 8.1% trehalose, 0.1% polysorbate 80;
- Control Formulation 4 (a formulation having the same composition as Amgevita®, except that 100 mg/ml of adalimumab was included instead of 50 mg/ml of adalimumab): 100 mg/ml of adalimumab, pH 5.2, 10 mM acetate, 9.0% sucrose, 0.1% polysorbate 80;
- Control Formulation 5 (a formulation having the same composition as Humira® (100 mg/mL)): 100 mg/ml of adalimumab, pH 5.2, 4.2%(w/v) mannitol, 0.1%(w/v) polysorbate 80.

2-2-2. Stability of formulation under heat stress conditions

%HMW

The experimental formulations and control formulations prepared in Example 2-2-1 were stored at 40±2 °C for 4 weeks, and then high molecular weight(%) (%HMW) of the protein in each formulation at 0th week, 1st week, 2nd week, and 4th week was measured by SE-HPLC analysis according to Reference Example 1. Further, changes in %HMW (Δ%HMW = (%HMW at corresponding week) - (%HMW at 0th week)) for 1 week, 2 weeks, and 4 weeks were calculated using the measured %HMW values.

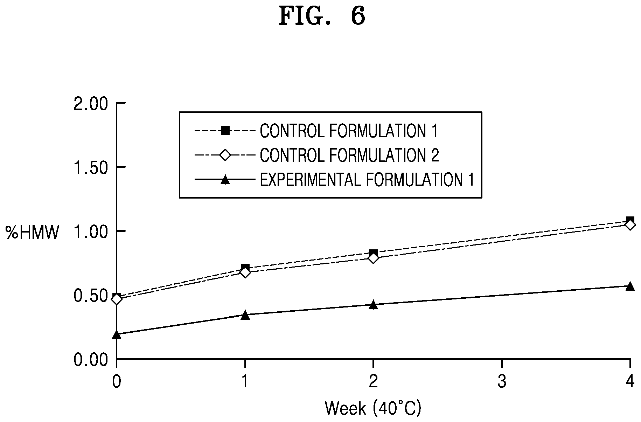

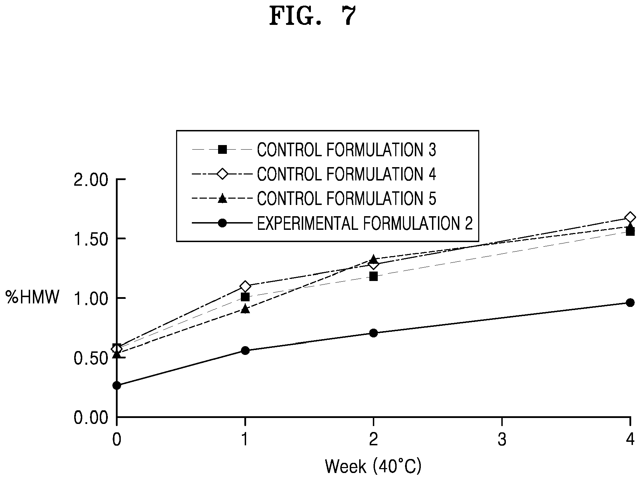

Average values (N=3) of the obtained results of %HMW and Δ%HMW are shown in Table 13 and FIG. 6 (a formulation having a protein concentration of 50 mg/mL) and Table 14 and FIG. 7 (a formulation having a protein concentration of 100 mg/mL):

[Table 13]

| Formulation | | %HMW | | | | Δ%HMW | | |
|---|---|---|---|---|---|---|---|---|
| | | 0 Wk | 1 Wk | 2 Wk | 4 Wk | 1 Wk | 2 Wk | 4 Wk |
| Experimental formulation 1 | Average | 0.20 | 0.35 | 0.43 | 0.57 | 0.15 | 0.23 | 0.37 |
| | SD (N=3) | 0.00 | 0.01 | 0.01 | 0.01 | | | |
| Control formulation 1 | Average | 0.48 | 0.70 | 0.82 | 1.07 | 0.22 | 0.34 | 0.60 |
| | SD (N=3) | 0.01 | 0.04 | 0.05 | 0.06 | | | |
| Control formulation 2 | Average | 0.46 | 0.68 | 0.79 | 1.05 | 0.24 | 0.32 | 0.59 |
| | SD (N=3) | 0.02 | 0.03 | 0.03 | 0.05 | | | |

[Table 14]

| Formulation | | %HMW | | | | Δ%HMW | | |
|---|---|---|---|---|---|---|---|---|
| | | 0 Wk | 1 Wk | 2 Wk | 4 Wk | 1 Wk | 2 Wk | 4 Wk |
| Experimental formulation 2 | Average | 0.27 | 0.55 | 0.69 | 0.95 | 0.27 | 0.41 | 0.68 |
| | SD (N=3) | 0.01 | 0.03 | 0.03 | 0.02 | | | |
| Control formulation 3 | Average | 0.59 | 1.01 | 1.19 | 1.56 | 0.43 | 0.60 | 0.98 |
| | SD (N=3) | 0.05 | 0.05 | 0.03 | 0.06 | | | |
| Control formulation 4 | Average | 0.58 | 1.10 | 1.28 | 1.68 | 0.52 | 0.72 | 1.10 |
| | SD (N=3) | 0.02 | 0.01 | 0.01 | 0.04 | | | |

(continued)

| Formulation | | %HMW | | | | Δ%HMW | | |
|---|---|---|---|---|---|---|---|---|
| | | 0 Wk | 1 Wk | 2 Wk | 4 Wk | 1 Wk | 2 Wk | 4 Wk |
| Control formulation 5 | Average | 0.53 | 0.90 | 1.33 | 1.60 | 0.37 | 0.80 | 1.07 |
| | SD (N=3) | 0.01 | 0.01 | 0.02 | 0.05 | | | |

The results of Tables 13 and 14 and FIGS. 6 and 7 confirmed that experimental formulations including histidine and polysorbate 20, wherein the protein concentration was 50 mg/mL or 100 mg/mL, showed remarkably low %HMW and Δ%HMW at each measurement point, as compared with control formulations free of histidine and polysorbate 20, indicating that experimental formulations had excellent stability.

%Acidic

The experimental formulations and control formulations prepared in Example 2-2-1 were stored at 40±2 °C for 4 weeks, and then %Acidic (a content ratio of acidic variants) of the protein in each formulation at 0th week, 2nd week, and 4th week was measured by CEX-HPLC analysis of Reference Example 4 (control formulations 1, 2, 3, and 4) or by icIEF analysis of Reference Example 5 (experimental formulations 1 and 2, and control formulation 5). Further, changes in %Acidic (Δ%Acidic = (%Acidic at corresponding week) - (%Acidic at 0th week)) were calculated using the measured %Acidic values.

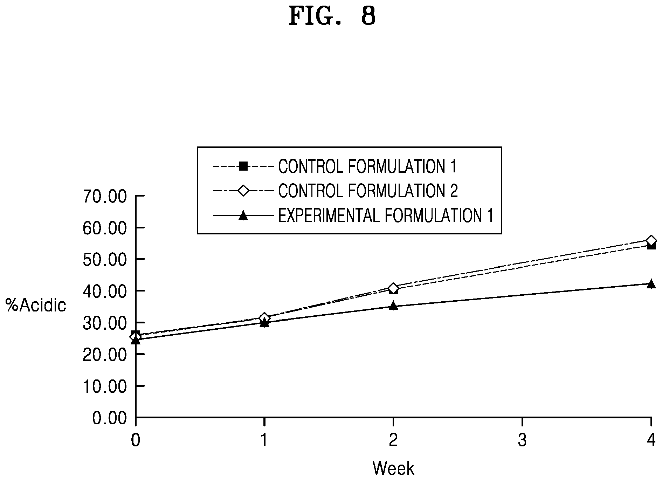

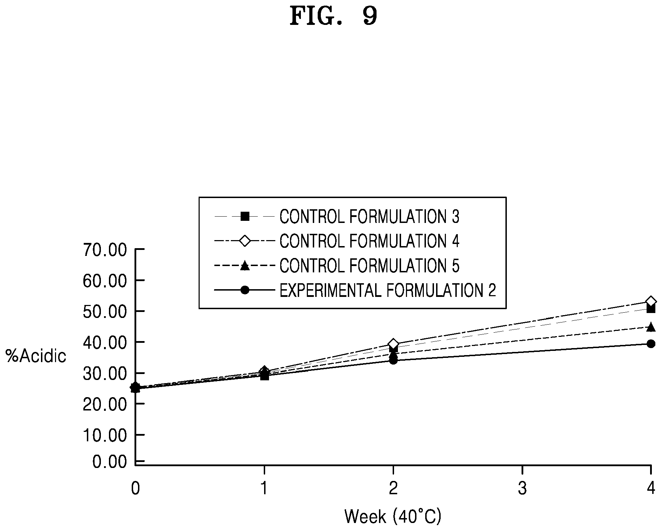

The obtained results (average value: N=3) of %Acidic and Δ%Acidic are shown in Table 15 and FIG. 8 (a formulation having a protein concentration of 50 mg/mL) and Table 16 and FIG. 9 (a formulation having a protein concentration of 100 mg/mL):

[Table 15]

| Formulation | | %Acidic | | | Δ%Acidic | |
|---|---|---|---|---|---|---|
| | | 0 Wk | 2 Wk | 4 Wk | 2 Wk | 4 Wk |
| Experimental formulation 1 | Average | 25.32 | 35.33 | 42.40 | 10.01 | 17.08 |
| | SD (N=3) | 0.62 | 1.64 | 0.66 | | |
| Control formulation 1 | Average | 26.50 | 40.65 | 55.02 | 14.16 | 28.52 |
| | SD (N=3) | 0.89 | 0.75 | 1.34 | | |
| Control formulation 2 | Average | 25.93 | 41.35 | 56.23 | 15.42 | 30.30 |
| | SD (N=3) | 0.30 | 0.61 | 1.00 | | |

[Table 16]

| Formulation | | %Acidic | | | Δ%Acidic | |
|---|---|---|---|---|---|---|
| | | 0 Wk | 2 Wk | 4 Wk | 2 Wk | 4 Wk |
| Experimental formulation 2 | Average | 24.79 | 33.79 | 39.28 | 8.99 | 14.49 |
| | SD (N=3) | 0.41 | 0.41 | 0.80 | | |
| Control formulation 3 | Average | 25.26 | 38.37 | 51.46 | 13.11 | 26.20 |
| | SD (N=3) | 1.39 | 0.78 | 1.23 | | |
| Control formulation 4 | Average | 24.58 | 39.19 | 52.84 | 14.61 | 28.26 |
| | SD (N=3) | 0.44 | 0.30 | 0.62 | | |
| Control formulation 5 | Average | 25.08 | 35.87 | 45.07 | 10.78 | 19.98 |
| | SD (N=3) | 1.99 | 0.78 | 0.92 | | |

The results of Tables 15 and 16 and FIGS. 8 and 9 confirmed that experimental formulations including histidine and polysorbate 20, wherein the protein concentration was 50 mg/mL or 100 mg/mL, showed remarkably low %Acidic and Δ%Acidic at each measurement point, as compared with control formulations free of histidine and polysorbate 20, indicating that experimental formulations had excellent stability.

Example 3. Optimization of Surfactant (polysorbate 20) Concentration

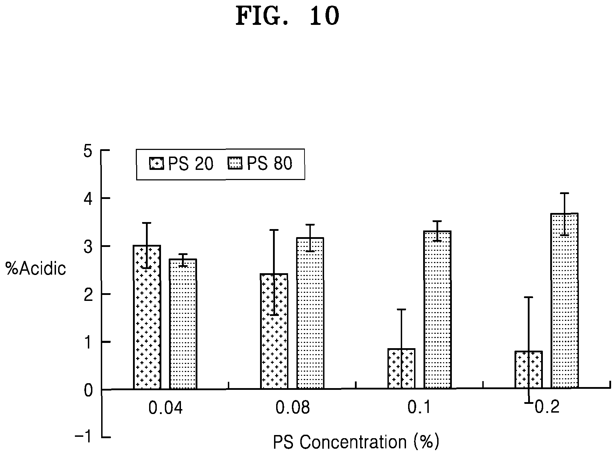

%Acidic of the protein in each formulation prepared in Example 2-1 under freeze-thaw conditions (Freeze/thaw, 5 cycles; FT5; each cycle: 'frozen at -70 °C±10 °C for 18 hours or longer' + 'thawed at room temperature (25 °C) for 1 hour or longer') was measured by icIEF analysis according to Reference Example 5. Further, changes (Δ%Acidic) in %Acidic before and after FT5 were calculated using the measured %Acidic values [Δ%Acidic = (FT5 %Acidic) - (Initial %Acidic)]. The experiment was repeated three times (n=3), and the average values of the obtained results are shown in the following Table 17, and FIG. 10:

[Table 17]

| %Acidic under FT5 (Freeze/thaw, 5 cycles) conditions | | | | |
| --- | --- | --- | --- | --- |
| Formulation | | %Acidic | | Δ%Acidic [(FT5 %Acidic) - (Initial %Acidic)] |
| | | Initial | FT5 | |
| 0.04% PS 20 | Average | 23.92 | 26.91 | 2.99 |
| | SD (N=3) | 0.28 | 0.22 | 0.46 |
| 0.08% PS 20 | Average | 24.40 | 26.82 | 2.42 |
| | SD (N=3) | 0.46 | 0.43 | 0.88 |
| 0.1% PS 20 | Average | 24.79 | 25.62 | 0.83 |
| | SD (N=3) | 0.34 | 0.85 | 0.83 |
| 0.2% PS 20 | Average | 24.93 | 25.72 | 0.79 |
| | SD (N=3) | 0.39 | 0.79 | 1.09 |
| 0.04% PS 80 | Average | 24.22 | 26.92 | 2.70 |
| | SD (N=3) | 0.31 | 0.36 | 0.10 |
| 0.08% PS 80 | Average | 23.82 | 26.97 | 3.15 |
| | SD (N=3) | 0.32 | 0.05 | 0.28 |
| 0.1% PS 80 | Average | 23.64 | 26.94 | 3.29 |
| | SD (N=3) | 0.34 | 0.19 | 0.20 |
| 0.2% PS 80 | Average | 23.74 | 27.37 | 3.63 |
| | SD (N=3) | 0.58 | 0.31 | 0.43 |

As shown in Table 17 and FIG. 10, the formulations including polysorbate 20 as a surfactant showed overall low Δ%Acidic values, as compared with formulations including polysorbate 80. In particular, when the concentration was 0.08% or more, the formulations including polysorbate 20 showed remarkably low Δ%Acidic values of about 2.5% or less, as compared with the formulations including polysorbate 80. In detail, it was confirmed that when the concentration of the surfactant was in the range of 0.08% or more, the Δ%Acidic values of the formulations including polysorbate 20 tend to decrease with increasing concentration of polysorbate 20, whereas the Δ%Acidic values of the formulations including polysorbate 80 tend to increase with increasing concentration of polysorbate 80. These results indicate that the formulations including polysorbate 20 had excellent stability, as compared with the formulations including polysorbate 80, and in particular, when the concentration of polysorbate 20 was 0.08% or more, the stability was particularly excellent.

Example 4. Quality Evaluation of Antibody-Containing Product - Temperature Excursion Test

During commercialization, storage, distribution, and clinical trials of the formulations containing the antibody, temperature excursions outside the recommended temperature for the formulations may occur, which may impede proper prescription. In this exemplary embodiment, to examine storage stability of the antibody (adalimumab) formulations, which were confirmed in Examples 1 to 3 to have excellent stability, formulation stability of pre-filled syringe (PFS) products, which were exposed to high temperature and low temperature conditions for a short period of time, was evaluated.

Compositions of the antibody formulations (samples) used in this exemplary embodiment are as follows: 50 mg/ml of adalimumab, pH 5.2, 10 mM Na-citrate, 59 mM histidine, 2.5%(w/v) sorbitol, 0.08%(w/v) polysorbate 20.

Pre-filled syringes with the antibody (adalimumab) formulation in a single dose (0.8 mL) were prepared (n=132), and 3 cycles of freeze/thaw were performed using the pre-filled syringes. Each cycle was performed by exposure to a high temperature (30±2 °C and relative humidity (RH) of 65±5%) for 48 hours, and then exposure to a low temperature (-5±3 °C) for 48 hours (3 cycles: high temperature exposure (30±2 °C) for a total of 144 hours and low temperature exposure (-5±3 °C) for a total of 144 hours). Thereafter, analysis was performed by methods of evaluating various items as follows: appearance, pH, protein concentration, container closure integrity, protein aggregation rate (%HMW), purity, charge variants (e.g., %Acidic, %Main, %Basic), oxidation of amino acid residues, endotoxin, particulates, and biological activity.

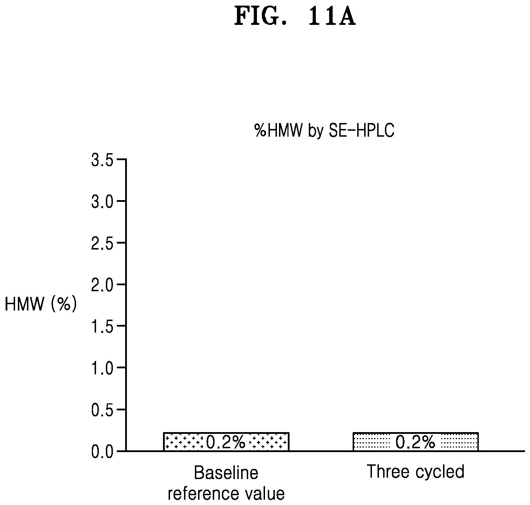

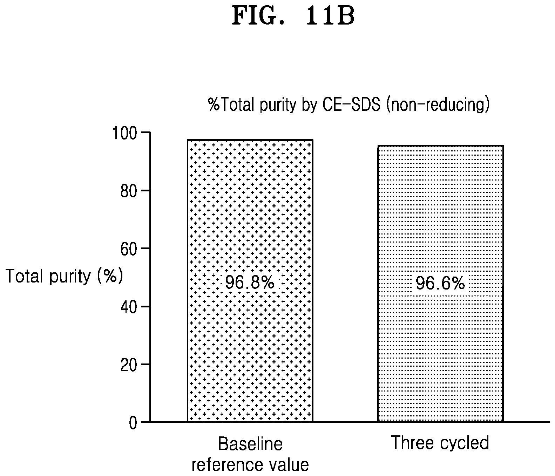

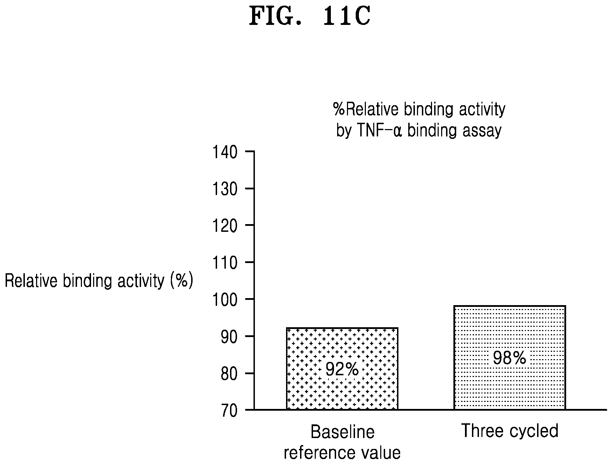

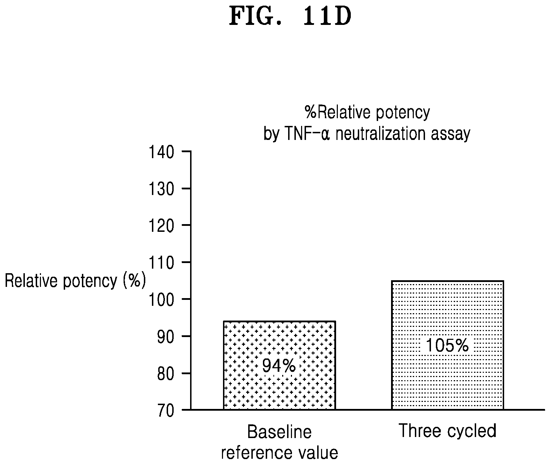

The effects of the freeze-thaw treatment (3 thermal cycles) on quality of the antibody formulation products (pre-filled syringes), as compared with those of a baseline (antibody formulation preserved at 5 °C), are shown in Table 18 below, and among them, %HMW, %Total purity, TNFα binding activity, and TNFα neutralizing activity are shown in FIGS. 11A to 11D (11A: %HMW, 11B: %Total purity, 11C: TNFα binding activity, and 11D: TNFα neutralizing activity), respectively.

[Table 18]

| Quality of Antibody Formulation Product (3 cycled sample) Under Extreme Temperature Cycling Conditions | | | |
|---|---|---|---|
| Test Item | Test method | Baseline | Temperature Cycle 3 |
| Color | Reference Example 6.1 | Colourless | B8 ≤ Sample < B7 |
| Clarity | Reference Example 6.1 (Nephelometric Turbidity Unit; NTU) | 18 NTU | 17 NTU |
| Visible particles | Reference Example 6.1 | Practically free from particles | Practically free from particles |
| pH | Reference Example 6.2 | 5.3 | 5.3 |
| Protein concentration (mg/mL) | Reference Example 6.3 | 51.6 | 49.7 |
| Polymer aggregate (impurity) (%HMW) | Reference Example 6.4 and Reference Example 1 | 0.2% | 0.2% |
| Total purity | Reference Example 6.5 (CE-SDS; Capillary Electrophoresis-Sodium Dodecyl Sulfate, Non-reducing) | 96.8% | 96.6% |
| Single highest impurity | | 2.1 | 2.0 |
| Main peak | icIEF (Reference Example 6.6 and Reference Example 5) | 8.6 | 8.6 |
| %Acidic | | 21.8 | 25.0 |
| %Main | | 67.1 | 64.5 |
| %Basic | | 11.2 | 10.5 |
| TNFα binding activity (%relative binding activity) | Competitive Binding Assay (FRET) (Reference Example 6.7) | 92% | 98% |
| TNFα neutralizing activity (%relative potency) | Cell-based, NF-κB Reporter Gene assay (Reference Example 6.8) | 94% | 105% |

(continued)

| Quality of Antibody Formulation Product (3 cycled sample) Under Extreme Temperature Cycling Conditions | | | |
|---|---|---|---|
| Test Item | Test method | Baseline | Temperature Cycle 3 |
| Number of particles having 10 μm or more (particles /syringe) | Reference Example 6.9 | 1521 | 1494 |
| Number of particles having 25 μm or more (particles /syringe) | | 15 | 18 |
| Number of particles having 2 μm or more (particles /syringe) | | 12217 | 13111 |
| Number of particles having 5 μm or more (particles /syringe) | | 6257 | 6882 |
| Number of particles having 8 μm or more (particles /syringe) | | 2476 | 2579 |
| Endotoxin | Endotoxin Units per ml (EU/mL) (Reference Example 6.10) | < 5 EU/mL | < 5 EU/mL |
| %acidic | CEX-HPLC (Reference Example 6.6 and Reference Example 4) | 23.5 | 24.0 |
| %main | | 67.2 | 65.2 |
| %basic | | 9.3 | 10.9 |
| %Oxidation of heavy chain Met34 | Reference Example 3 | 0.6 | 0.6 |
| %Oxidation of heavy chain Met83 | | 0.3 | 0.4 |
| %Oxidation of heavy chain Met256 | | 5.8 | 4.7 |
| %Oxidation of heavy chain Met432 | | Not detected | Not detected |
| %Oxidation of light chain Met4 | | 0.2 | 0.5 |

As shown in Table 18 and FIGS. 11A to 11D, the pre-filled syringes which were filled with the antibody formulation and treated with 3 cycles (each cycle: high temperature (30±2 °C) exposure and low temperature (-5±3 °C) exposure), as tested in the present exemplary embodiment, showed no significant changes in four critical quality attributes (CQA) regarding %HMW by SE-HPLC, %Total purity by non-reducing CE-SDS, %relative binding activity by TNF-α binding assay, and %relative potency by a cell-based TNF-α neutralization assay, as compared with the baseline, and the results met stability acceptance criteria.

In addition, charge variants of antibody, oxidation degree of amino acid residue, endotoxin levels, appearance (clarity, visible particles, etc.), pH, protein concentration, and subvisible particles in the antibody formulation products (prefilled syringes) tested in the present exemplary embodiment also showed no significant changes, as compared with those of the reference, and the results met stability acceptance criteria.

Further, none of the tested pre-filled syringes showed closure breaches of the container.

As described above, the antibody formulations tested in the present exemplary embodiment showed excellent stability inside the products even after exposed to several times of freeze-thaw cycles (thermal cycles) immediately after commercialization. These results allow prediction of the effects of temperature excursions during shipment or preservation of the antibody formulation products, and thus are expected to contribute to prescribing the antibody.

Example 5. Evaluation of Storage Stability at Room Temperature - Patient Convenience Stability Study

With respect to the formulation samples of Example 4 preserved for 48 months under long-term storage conditions (5±3 °C), physical properties of Table 19 were measured at the time point of 0 day, 15 days, and 30 days while preserved for 30 days under room temperature conditions (30±2 °C and RH of 60±5%). Detailed measurement methods are as performed in Example 4.

[Table 19]

| Stability results under room-temperature storage conditions (30 ± 2 °C/ RH of 65 ± 5%) | | | | | |
|---|---|---|---|---|---|
| Test Item | | Time Point | | | |
| | | 0 Day | 15 Days | 30 Days | |
| Appearance | Color | B8 < sample < B7 | N/A | B9 < sample < B8 | |
| | Clarity | 17 | | 17 | |
| | Visible particles | Practically free from particles | | Practically free from particles | |
| pH | | 5.3 | | 5.3 | |
| Protein content ($A_{280}$) | | 50.1 | | 50.7 | |
| Size exclusion chromatography (SE-HPLC) | | 0.7 | 0.8 | 0.9 | |
| CE-SDS (non-reducing) | | 96.9 | 95.6 | 96.1 | |
| | | 1.9 | 1.9 | 1.7 | |
| icIEF | | 29 | 31 | 33 | |
| | | 62 | 59 | 56 | |
| | | 9 | 10 | 11 | |
| Competitive inhibition binding assay (CIBA) of TNF-$\alpha$ by FRE | | 102 | 100 | 106 | |
| Analysis of TNF-$\alpha$ neutralization by NF-$\kappa$B reporter gene | | 93 | 100 | 86 | |
| Particulate matter | | 6 | N/S | 12 | |
| | | 1,231 | | 923 | |
| N/A: not applicable | | | | | |

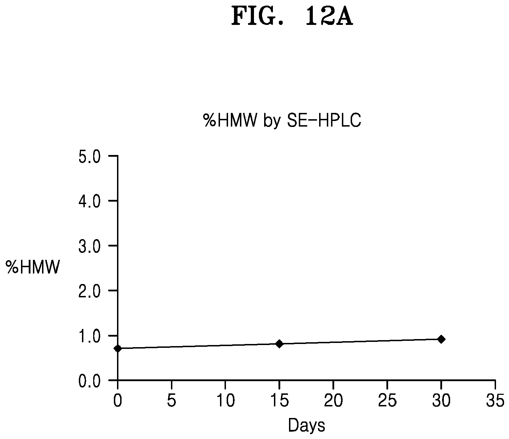

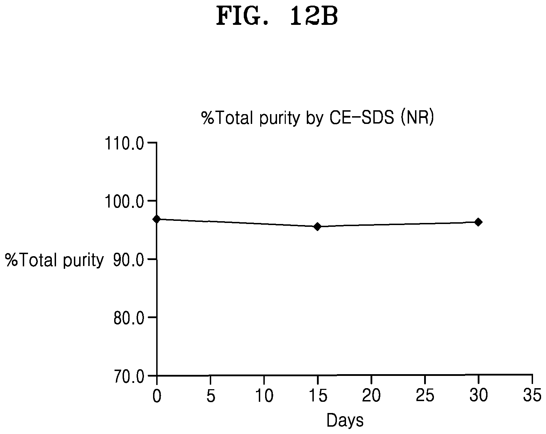

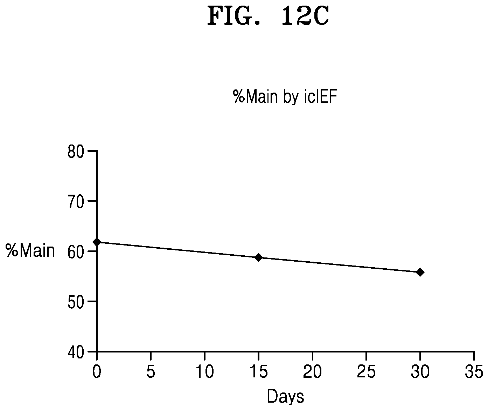

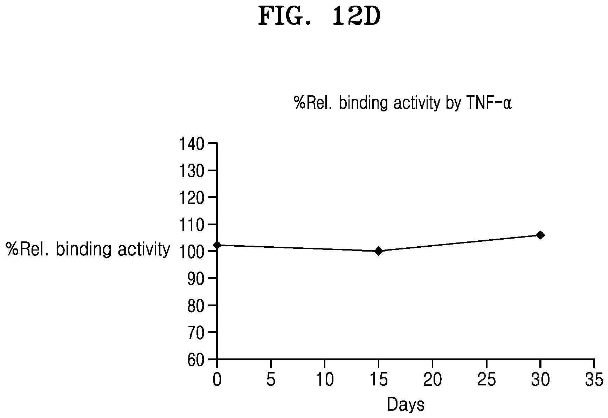

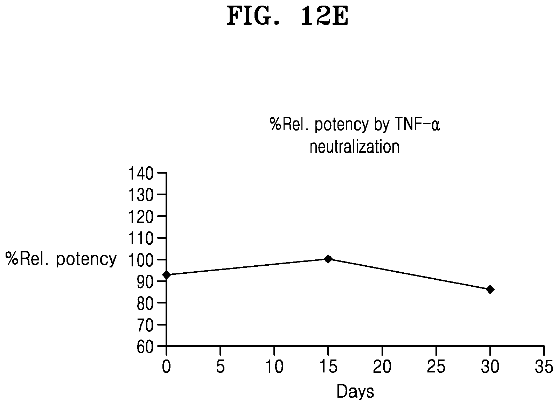

As shown in Table 19 and FIGS. 12A to 12E, the antibody formulation samples tested in the present exemplary embodiment were tested under room temperature conditions (30±2 °C and RH of 60±5%) for 30 days, after being preserved for 48 months under long-term storage conditions (5±3 °C), and as a result, they showed no significant changes in four critical quality attributes (CQA) regarding %HMW by SE-HPLC, %Total purity by non-reducing CE-SDS, %relative binding activity by TNF-$\alpha$ binding assay, and %relative potency by a cell based TNF-$\alpha$ neutralization assay, as compared with the baseline (0 day), and the results met stability acceptance criteria.

Further, they also showed no significant changes in %Main by icIEF, as compared with the baseline (0 day), and the results met stability acceptance criteria.

Accordingly, the pharmaceutical compositions of the present disclosure exhibit significantly improved patient convenience and a longer lifetime characteristic during commercialization, as compared with the original and biosimilars thereof.

**Claims**

**1.** A pharmaceutical composition comprising:

an anti-TNFa antibody, histidine, and polysorbate 20,
wherein pH is 5.0 to 5.5, and
the pharmaceutical composition has one or more selected from the following characteristics;

> (i) improvement in photostability, as compared with a histidine-free composition; and
> (ii) improvement in thermal stability, freeze-thaw stability, or both thermal stability and freeze-thaw stability, as compared with a composition comprising polysorbate 80 instead of polysorbate 20.

**2.** The pharmaceutical composition of claim 1,
wherein the improvement in photostability is reduction in an oxidation rate of an amino acid residue and/or an aggregate content (%High Molecular Weight; %HMW) in the antibody under light-exposed conditions.

**3.** The pharmaceutical composition of claim 1 or 2,
wherein the pharmaceutical composition has a low oxidation rate of an amino acid residue and/or a low aggregate content (%high molecular weight (%HMW)) in the antibody under light-exposed conditions, as compared with the histidine-free composition.

**4.** The pharmaceutical composition of any one of the preceding claims, wherein the light-exposed conditions are 1.2 million lux hours or more and 200 watt hours/square meter or more.

**5.** The pharmaceutical composition of any one of the preceding claims, wherein the amino acid residue of the antibody is a methionine residue at position 256 of a heavy chain of the antibody.

**6.** The pharmaceutical composition of any one of the preceding claims, wherein the oxidation rate of the amino acid residue is measured as an oxidation rate ($\%Met_{256}$) of a methionine residue at position 256 of a heavy chain of the antibody under light-exposed conditions, and %Met256 is 64% or less.

**7.** The pharmaceutical composition of any one of the preceding claims, wherein the oxidation rate of the amino acid residue is measured as an oxidation rate ($\%Met_{256}$) of a methionine residue at position 256 of a heavy chain of the antibody under light-exposed conditions, and $\%Met_{256}$ shows 15% or higher reduction, as compared with $\%Met_{256}$ of the histidine-free composition under light-exposed conditions.

**8.** The pharmaceutical composition of any one of the preceding claims, wherein a change in the oxidation rate of the amino acid residue is measured as a difference ($\Delta\%Met_{256}$) between an oxidation rate ($\%Met_{256}$) of a methionine residue at position 256 of a heavy chain of the antibody under light-exposed conditions and %Met256 of a base group or a dark control, and $\Delta\%Met_{256}$ is 55% or less.

**9.** The pharmaceutical composition of any one of the preceding claims,

wherein a change in the oxidation rate of the amino acid residue is measured as a difference ($\Delta\%Met_{256}$) between an oxidation rate ($\%Met_{256}$) of a methionine residue at position 256 of a heavy chain of the antibody under light-exposed conditions and %Met256 of a base group or a dark control, and
$\Delta\%Met_{256}$ shows 10% or higher reduction, as compared with that of the histidine-free composition.

**10.** The pharmaceutical composition of any one of the preceding claims, wherein the aggregate content (%HMW) is measured as an aggregate content under light-exposed conditions, and %HMW is 15% or less.

**11.** The pharmaceutical composition of any one of the preceding claims, wherein the aggregate content is measured as an aggregate content under light-exposed conditions, and %HMW shows 5% or higher reduction, as compared with that of the histidine-free composition under light-exposed conditions.

**12.** The pharmaceutical composition of any one of the preceding claims, wherein a change in the aggregate content is measured as a difference ($\Delta\%HMW$) between the aggregate content in a light-exposed group and the aggregate content of a base group or a dark control, and $\Delta\%HMW$ is 15% or less.

**13.** The pharmaceutical composition of any one of the preceding claims, wherein a change in the aggregate content is measured as a difference ($\Delta\%HMW$) between the aggregate content under light-exposed conditions and the aggregate content of a base group or a dark control, and $\Delta\%HMW$ shows 3% or higher reduction, as compared with that of the histidine-free composition.

**14.** The pharmaceutical composition of any one of the preceding claims, wherein the histidine functions as an anti-oxidant agent, an anti-aggregation agent, or both an anti-oxidant agent and an anti-aggregation agent.

15. The pharmaceutical composition of any one of the preceding claims, wherein the improvement of thermal stability is measured as an aggregate content (%HMW) during preservation at $40\pm2$ °C for 2 weeks.

16. The pharmaceutical composition of any one of the preceding claims, wherein the pharmaceutical composition has a low aggregate content (%HMW) after being preserved at $40\pm2$ °C for 2 weeks, as compared with a composition comprising polysorbate 80 instead of polysorbate 20.

17. The pharmaceutical composition of any one of the preceding claims, wherein the pharmaceutical composition has an aggregate content (%HMW) of 0.70% or less, after being preserved at $40\pm2$ °C for 2 weeks.

18. The pharmaceutical composition of any one of the preceding claims, wherein the improvement in freeze-thaw stability is measured as an aggregate content (%HMW) after 5 cycles of freeze-thaw at $-70\pm10$ °C/25 °C.

19. The pharmaceutical composition of any one of the preceding claims, wherein the pharmaceutical composition has a low aggregate content (%HMW) after 5 cycles of freeze-thaw at $-70\pm10$ °C/25 °C, as compared with a composition comprising polysorbate 80 instead of polysorbate 20.

20. The pharmaceutical composition of any one of the preceding claims, wherein the pharmaceutical composition has an aggregate content (%HMW) of 0.49% or less, after 5 cycles of freeze-thaw at $-70\pm10$ °C/25 °C.

21. The pharmaceutical composition of any one of the preceding claims, wherein the improvement in freeze-thaw stability is measured as an acidic variant content (%Acidic) after 5 cycles of freeze-thaw at $-70\pm10$ °C/25 °C.

22. The pharmaceutical composition of any one of the preceding claims, wherein the pharmaceutical composition has a low acidic variant content (%Acidic) after 5 cycles of freeze-thaw at $-70\pm10$ °C/25 °C or a low %Acidic difference ($\Delta$%Acidic) before and after 5 cycles of freeze-thaw at $-70\pm10$ °C/25 °C, as compared with a composition comprising polysorbate 80 instead of polysorbate 20.

23. The pharmaceutical composition of any one of the preceding claims, wherein the pharmaceutical composition has an acidic variants content (%Acidic) of 27% or less after 5 cycles of freeze-thaw at $-70\pm10$ °C/25 °C.

24. The pharmaceutical composition of any one of the preceding claims, wherein the pharmaceutical composition exhibits a 0.1% or higher reduction in acidic variant content (%Acidic) after 5 cycles of freeze-thaw at $-70\pm10$ °C/25 °C, as compared with a composition comprising polysorbate 80 instead of polysorbate 20.

25. The pharmaceutical composition of any one of the preceding claims, wherein a change ($\Delta$%Acidic) in %Acidic of the pharmaceutical composition is 2.5% or less, before and after 5 cycles of freeze-thaw at $-70\pm10$ °C/25 °C.

26. The pharmaceutical composition of any one of the preceding claims, wherein the pharmaceutical composition exhibits a 0.2% or higher reduction in change ($\Delta$%Acidic) in %Acidic, before and after 5 cycles of freeze-thaw at $-70\pm10$ °C/25 °C, as compared with a composition comprising polysorbate 80 instead of polysorbate 20.

27. The pharmaceutical composition of any one of the preceding claims, wherein the pharmaceutical composition has a preservation period of 28 days to 35 days at a temperature of $30\pm2$ °C, after being stored at a temperature of $5\pm3$ °C for 32 months to 52 months.

28. The pharmaceutical composition of any one of the preceding claims, wherein the pharmaceutical composition has a preservation period of 30 days at a temperature of $30\pm2$ °C, after being stored at a temperature of $5\pm3$ °C for 36 months to 48 months.

29. The pharmaceutical composition of any one of the preceding claims, wherein the pharmaceutical composition, when preserved at a temperature of $30\pm2$ °C for 28 days to 35 days after being stored at a temperature of $5\pm3$ °C for 32 months to 52 months, has one or more of the following characteristics:

   turbidity of 20 nephelometric turbidity units (NTU) or less,
   TNF$\alpha$ binding activity of about 90% or more,

TNFα neutralizing activity of about 80% or more,
total purity of 93% or more,
an aggregate content (%HMW) of 2% or less,
an acidic variant content (%Acidic) of 33% or less,
a main protein content (%Main) of 55% or more,
a basic variant content (%basic) of 14% or less,
in 0.8 mL of the pharmaceutical composition, the number of particles having an average particle diameter of 10 μm or more is 1500 or less,
in 0.8 mL of the pharmaceutical composition, the number of particles having an average particle diameter of 25 μm or more is 40 or less, and
an endotoxin content of 8 EU/mL or less.

30. The pharmaceutical composition of any one of the preceding claims, wherein the anti-TNFa antibody is adalimumab.

31. The pharmaceutical composition of any one of the preceding claims, wherein the anti-TNFa antibody is comprised at a concentration of about 50 mg/mL or about 100 mg/mL.

32. The pharmaceutical composition of any one of the preceding claims, wherein the histidine is comprised at a concentration of 50 mM to 89 mM.

33. The pharmaceutical composition of any one of the preceding claims, wherein the histidine is comprised at a concentration of 59 mM.

34. The pharmaceutical composition of any one of the preceding claims, wherein the pharmaceutical composition is free of amino acids other than histidine.

35. The pharmaceutical composition of any one of the preceding claims, wherein the polysorbate 20 is comprised at a concentration of more than 0.04%(w/v) and 0.2%(w/v) or less.

36. The pharmaceutical composition of any one of the preceding claims, wherein the polysorbate 20 is comprised at a concentration of about 0.08%(w/v).

37. The pharmaceutical composition of any one of the preceding claims, wherein the pharmaceutical composition is free of polysorbate 80.

38. The pharmaceutical composition of any one of the preceding claims, wherein the pharmaceutical composition further comprises one or more selected from the group consisting of sorbitol, mannitol, meglumine, trehalose, sucrose, maltose, lactose, glucose, xylitol, arabitol, erythritol, lactitol, maltitol, and inositol.

39. The pharmaceutical composition of any one of the preceding claims, wherein the pharmaceutical composition further comprises citrate.

40. The pharmaceutical composition of any one of the preceding claims, wherein the pharmaceutical composition further comprises one or more selected from the group consisting of phosphate, succinate, and acetate.

41. The pharmaceutical composition of claim 40,
wherein the pharmaceutical composition is free of citrate.

42. The pharmaceutical composition of any one of the preceding claims, wherein the pharmaceutical composition is free of one or more selected from the group consisting of ammonium chloride, ammonium sulfate, ammonium carbonate, ammonium nitrate, sodium chloride, sodium sulfate, potassium chloride, sodium hydroxide, potassium hydroxide, and ethylenediaminetetraacetic acid (EDTA).

43. A container comprising:

the pharmaceutical composition of any one of claims 1 to 42,
wherein the pharmaceutical composition has stability against temperature changes, after 3 cycles each consisting of exposure to a high temperature of 30±2 °C for 48 hours and exposure to a low temperature of -5±3 °C for 48 hours.

**44.** The container of claim 43,
wherein the stability against temperature changes is determined by one or more selected from the group consisting of TNFα-binding activity, TNFα-neutralizing activity, the number of subvisible particles, oxidation of amino acid residue, clarity, pH, protein concentration, protein aggregation rate, purity, charge variants, and endotoxin.

**45.** The container of claim 43 or 44, wherein the pharmaceutical composition, after 3 cycles consisting of exposure to a high temperature of 30±2 °C for 48 hours and exposure to a low temperature of -5±3 °C for 48 hours, has one or more selected from the following characteristics:

TNFα binding activity of about 90% or more,
TNFα neutralizing activity of about 90% or more,
an oxidation rate of methionine at position 34 of a heavy chain of the antibody of about 1.1% or less,
an oxidation rate of methionine at position 83 of a heavy chain of the antibody of about 0.9% or less,
an oxidation rate of methionine at position 256 of a heavy chain of the antibody of about 6.0% or less,
an oxidation rate of methionine at position 432 of a heavy chain of the antibody of about 0.5% or less,
an oxidation rate of methionine at position 4 of a light chain of the antibody of about 0.9% or less,
turbidity of 20 nephelometric turbidity units (NTU) or less,
total purity of 94%(w/v) or more,
in 0.8 mL of the pharmaceutical composition, the number of particles having an average particle diameter of 10 μm or more is 2500 or less,
in 0.8 mL of the pharmaceutical composition, the number of particles having an average particle diameter of 25 μm or more is 100 or less, and
an endotoxin content of 8 EU/mL or less.

**46.** The container of any one of claims 40 to 42,
wherein the container comprises a vial, a pre-filled syringe (PFS), or a pre-filled pen (PFP).

# FIG. 1

# FIG. 2A

# FIG. 2B

# FIG. 2C

# FIG. 3

FIG. 4

# FIG. 5

# FIG. 6

# FIG. 7

# FIG. 8

# FIG. 9

# FIG. 10

# FIG. 11A

%HMW by SE-HPLC

# FIG. 11B

%Total purity by CE-SDS (non-reducing)

# FIG. 11C

%Relative binding activity
by TNF-α binding assay

Relative binding activity (%)

# FIG. 11D

%Relative potency
by TNF-α neutralization assay

# FIG. 12A

%HMW by SE-HPLC

# FIG. 12B

%Total purity by CE-SDS (NR)

# FIG. 12C

%Main by iclEF

# FIG. 12D

%Rel. binding activity by TNF-α

# FIG. 12E

%Rel. potency by TNF-α
neutralization

## INTERNATIONAL SEARCH REPORT

International application No.

**PCT/KR2021/002983**

### A. CLASSIFICATION OF SUBJECT MATTER

**A61K 39/395**(2006.01)i; **A61K 9/08**(2006.01)i; **A61K 47/22**(2006.01)i; **A61K 47/26**(2006.01)i; **A61K 47/12**(2006.01)i; **C07K 16/24**(2006.01)i

According to International Patent Classification (IPC) or to both national classification and IPC

### B. FIELDS SEARCHED

Minimum documentation searched (classification system followed by classification symbols)

A61K 39/395(2006.01); A61K 47/10(2006.01); A61K 47/14(2006.01); A61K 47/18(2006.01); A61K 47/22(2006.01); A61K 47/26(2006.01); A61P 37/00(2006.01); C07K 16/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 항-TNFα 항체(anti-TNFα antibody), 히스티딘(histidine), 폴리소르베이트 20(polysorbate 20), 안정성(stability)

### C. DOCUMENTS CONSIDERED TO BE RELEVANT

| Category* | Citation of document, with indication, where appropriate, of the relevant passages | Relevant to claim No. |
|---|---|---|
| X | KR 10-2018-0097471 A (CELLTRION, INC.) 31 August 2018 (2018-08-31) See claims 1, 2 and 10; paragraphs [0048], [0084]-[0086], [0234] (example 3), [0236], [0238]-[0241], [0247]-[0249], [0251]-[0253], [0255]-[0257] and [0267]-[0269]; and tables 2, 4-6 and 9. | 1-3 |
| A | US 2019-0070294 A1 (COHERUS BIOSCIENCES, INC.) 07 March 2019 (2019-03-07) See claims 1, 3 and 5; paragraphs [0026], [0098], [0180], [0229] and [0298]; and tables A, B-1 and G. | 1-3 |
| A | KR 10-2016-0068946 A (MEDIMMUNE, LLC) 15 June 2016 (2016-06-15) See abstract; claims 1 and 12; and paragraphs [0146], [0150] and [0153]. | 1-3 |
| A | EP 3563867 A1 (JOINT STOCK COMPANY "BIOCAD") 06 November 2019 (2019-11-06) See claims 1, 2, 5, 9, 20 and 25; and paragraphs [0029] and [0030]. | 1-3 |

☑ Further documents are listed in the continuation of Box C.  ☑ See patent family annex.

| | |
|---|---|
| *    Special categories of cited documents: | "T"  later document published after the international filing date or priority date and not in conflict with the application but cited to understand the principle or theory underlying the invention |
| "A"  document defining the general state of the art which is not considered to be of particular relevance | |
| "D"  document cited by the applicant in the international application | "X"  document of particular relevance; the claimed invention cannot be considered novel or cannot be considered to involve an inventive step when the document is taken alone |
| "E"  earlier application or patent but published on or after the international filing date | |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| **25 June 2021** | **25 June 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| | International application No. |
|---|---|
| | **PCT/KR2021/002983** |

**C.    DOCUMENTS CONSIDERED TO BE RELEVANT**

| Category* | Citation of document, with indication, where appropriate, of the relevant passages | Relevant to claim No. |
|---|---|---|
| A | KR 10-2017-0117166 A (SANOFI) 20 October 2017 (2017-10-20)<br>See entire document. | 1-3 |

Form PCT/ISA/210 (second sheet) (July 2019)

**INTERNATIONAL SEARCH REPORT**

International application No.

**PCT/KR2021/002983**

| Box No. II | Observations where certain claims were found unsearchable (Continuation of item 2 of first sheet) |
|---|---|

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **41,44**
because they relate to parts of the international application that do not comply with the prescribed requirements to such an extent that no meaningful international search can be carried out, specifically:

Claims 41 and 44 refer to claims violating PCT Rule 6.4(a), and thus the meaning thereof is unclear.

3. ☑ Claims Nos.: **4-40,42,43,45,46**
because they are dependent claims and are not drafted in accordance with the second and third sentences of Rule 6.4(a).

Form PCT/ISA/210 (continuation of first sheet) (July 2019)

| INTERNATIONAL SEARCH REPORT | | | | | | International application No. |
|---|---|---|---|---|---|---|
| Information on patent family members | | | | | | **PCT/KR2021/002983** |

| Patent document cited in search report | | | Publication date (day/month/year) | Patent family member(s) | | | Publication date (day/month/year) |
|---|---|---|---|---|---|---|---|
| KR | 10-2018-0097471 | A | 31 August 2018 | AU | 2017-287743 | A1 | 22 November 2018 |
| | | | | AU | 2017-287743 | B2 | 30 January 2020 |
| | | | | AU | 2017-287743 | C1 | 01 October 2020 |
| | | | | AU | 2020-201249 | A1 | 12 March 2020 |
| | | | | BR | 112018076377 | A2 | 26 March 2019 |
| | | | | CA | 3028238 | A1 | 04 January 2018 |
| | | | | CL | 2018003662 | A1 | 15 March 2019 |
| | | | | CN | 109310628 | A | 05 February 2019 |
| | | | | CO | 2018013689 | A2 | 18 January 2019 |
| | | | | CR | 20180599 | A | 09 April 2019 |
| | | | | CU | 20180154 | A7 | 06 August 2019 |
| | | | | DO | P2018000290 | A | 15 February 2019 |
| | | | | EA | 201892653 | A1 | 31 May 2019 |
| | | | | EC | SP18093651 | A | 31 January 2019 |
| | | | | EP | 3479819 | A1 | 08 May 2019 |
| | | | | JO | P20180125 | A1 | 30 January 2019 |
| | | | | JP | 2019-525902 | A | 12 September 2019 |
| | | | | KR | 10-2018-0003452 | A | 09 January 2018 |
| | | | | KR | 10-2021-0042052 | A | 16 April 2021 |
| | | | | KR | 10-2229274 | B1 | 18 March 2021 |
| | | | | MX | 2018015960 | A | 21 March 2019 |
| | | | | NI | 201800139 | A | 25 March 2019 |
| | | | | PE | 20190448 | A1 | 29 March 2019 |
| | | | | PH | 12018502670 | A1 | 07 October 2019 |
| | | | | SG | 11201811320 | A | 30 January 2019 |
| | | | | TN | 2018000443 | A1 | 15 June 2020 |
| | | | | TW | 201806617 | A | 01 March 2018 |
| | | | | UA | 122610 | C2 | 10 December 2020 |
| | | | | US | 2021-0000743 | A1 | 07 January 2021 |
| | | | | WO | 2018-004260 | A1 | 04 January 2018 |
| | | | | ZA | 201808476 | B | 26 August 2020 |
| US | 2019-0070294 | A1 | 07 March 2019 | AR | 092470 | A1 | 22 April 2015 |
| | | | | AU | 2013-312300 | A1 | 16 April 2015 |
| | | | | AU | 2018-208699 | A1 | 16 August 2018 |
| | | | | AU | 2020-202912 | A1 | 21 May 2020 |
| | | | | AU | 4294701 | A | 15 October 2001 |
| | | | | BR | 112015004984 | A2 | 04 July 2017 |
| | | | | CA | 2884182 | A1 | 13 March 2014 |
| | | | | CL | 2015000574 | A1 | 23 October 2015 |
| | | | | CN | 104768576 | A | 08 July 2015 |
| | | | | DK | 2892550 | T3 | 30 March 2020 |
| | | | | DO | P2015000051 | A | 31 May 2015 |
| | | | | EA | 029215 | B1 | 28 February 2018 |
| | | | | EA | 201590518 | A1 | 31 May 2016 |
| | | | | EA | 201791717 | A1 | 30 March 2018 |
| | | | | EC | SP15013227 | A | 29 January 2016 |
| | | | | EP | 2892550 | A2 | 15 July 2015 |
| | | | | EP | 2892550 | A4 | 13 April 2016 |
| | | | | EP | 2892550 | B1 | 18 December 2019 |
| | | | | EP | 3701968 | A1 | 02 September 2020 |

Form PCT/ISA/210 (patent family annex) (July 2019)

| | INTERNATIONAL SEARCH REPORT<br>Information on patent family members | International application No.<br>**PCT/KR2021/002983** | |
|---|---|---|---|

| Patent document<br>cited in search report | Publication date<br>(day/month/year) | Patent family member(s) | | | Publication date<br>(day/month/year) |
|---|---|---|---|---|---|
| | | ES | 2784861 | T3 | 01 October 2020 |
| | | HK | 1210601 | A1 | 29 April 2016 |
| | | HR | P20200434 | T1 | 24 July 2020 |
| | | HU | E049282 | T2 | 28 September 2020 |
| | | IL | 237583 | A | 29 October 2020 |
| | | JP | 2015-527402 | A | 17 September 2015 |
| | | JP | 2019-069993 | A | 09 May 2019 |
| | | JP | 2021-020930 | A | 18 February 2021 |
| | | JP | 6463268 | B2 | 30 January 2019 |
| | | JP | 6783845 | B2 | 11 November 2020 |
| | | KR | 10-2015-0047140 | A | 04 May 2015 |
| | | KR | 10-2021-0040470 | A | 13 April 2021 |
| | | LT | 2892550 | T | 10 April 2020 |
| | | MX | 2015003007 | A | 05 June 2015 |
| | | PE | 09642015 | A1 | 25 July 2015 |
| | | PE | 20150964 | A1 | 25 July 2015 |
| | | PE | 20191815 | A1 | 27 December 2019 |
| | | PL | 2892550 | T3 | 27 July 2020 |
| | | PT | 2892550 | T | 27 March 2020 |
| | | RS | 60226 | B1 | 30 June 2020 |
| | | SE | 0001087 | L | 28 September 2001 |
| | | SE | 519040 | C2 | 23 December 2002 |
| | | SG | 10201705668 | A | 30 August 2017 |
| | | SG | 11201501715 | A | 28 May 2015 |
| | | SI | 2892550 | T1 | 30 November 2020 |
| | | TW | 201424749 | A | 01 July 2014 |
| | | TW | 202042841 | A | 01 December 2020 |
| | | TW | I698253 | B | 11 July 2020 |
| | | US | 10155039 | B2 | 18 December 2018 |
| | | US | 10159732 | B2 | 25 December 2018 |
| | | US | 10159733 | B2 | 25 December 2018 |
| | | US | 10195275 | B2 | 05 February 2019 |
| | | US | 10207000 | B2 | 19 February 2019 |
| | | US | 10286071 | B2 | 14 May 2019 |
| | | US | 10286072 | B2 | 14 May 2019 |
| | | US | 10688183 | B2 | 23 June 2020 |
| | | US | 10716852 | B2 | 21 July 2020 |
| | | US | 10716853 | B2 | 21 July 2020 |
| | | US | 10716854 | B2 | 21 July 2020 |
| | | US | 10722579 | B2 | 28 July 2020 |
| | | US | 10772959 | B2 | 15 September 2020 |
| | | US | 10772960 | B2 | 15 September 2020 |
| | | US | 10780163 | B2 | 22 September 2020 |
| | | US | 10786566 | B2 | 29 September 2020 |
| | | US | 10799585 | B2 | 13 October 2020 |
| | | US | 2014-0186361 | A1 | 03 July 2014 |
| | | US | 2015-0182626 | A1 | 02 July 2015 |
| | | US | 2015-0190512 | A1 | 09 July 2015 |
| | | US | 2015-0190513 | A1 | 09 July 2015 |
| | | US | 2015-0191538 | A1 | 09 July 2015 |

Form PCT/ISA/210 (patent family annex) (July 2019)

<table>
<tr><td colspan="2" align="center">**INTERNATIONAL SEARCH REPORT**<br>Information on patent family members</td><td colspan="2">International application No.<br><br>**PCT/KR2021/002983**</td></tr>
<tr><td>Patent document<br>cited in search report</td><td>Publication date<br>(day/month/year)</td><td>Patent family member(s)</td><td>Publication date<br>(day/month/year)</td></tr>
<tr><td></td><td></td><td>US   2015-0191539  A1</td><td>09 July 2015</td></tr>
<tr><td></td><td></td><td>US   2016-0031982  A1</td><td>04 February 2016</td></tr>
<tr><td></td><td></td><td>US   2016-0039926  A1</td><td>11 February 2016</td></tr>
<tr><td></td><td></td><td>US   2016-0256545  A1</td><td>08 September 2016</td></tr>
<tr><td></td><td></td><td>US   2016-0256546  A1</td><td>08 September 2016</td></tr>
<tr><td></td><td></td><td>US   2016-0256547  A1</td><td>08 September 2016</td></tr>
<tr><td></td><td></td><td>US   2016-0263226  A1</td><td>15 September 2016</td></tr>
<tr><td></td><td></td><td>US   2016-0303233  A1</td><td>20 October 2016</td></tr>
<tr><td></td><td></td><td>US   2016-0303234  A1</td><td>20 October 2016</td></tr>
<tr><td></td><td></td><td>US   2016-0303235  A1</td><td>20 October 2016</td></tr>
<tr><td></td><td></td><td>US   2016-0304599  A1</td><td>20 October 2016</td></tr>
<tr><td></td><td></td><td>US   2016-0304600  A1</td><td>20 October 2016</td></tr>
<tr><td></td><td></td><td>US   2016-0304601  A1</td><td>20 October 2016</td></tr>
<tr><td></td><td></td><td>US   2017-0072054  A1</td><td>16 March 2017</td></tr>
<tr><td></td><td></td><td>US   2017-0312361  A1</td><td>02 November 2017</td></tr>
<tr><td></td><td></td><td>US   2018-0021433  A1</td><td>25 January 2018</td></tr>
<tr><td></td><td></td><td>US   2018-0028653  A1</td><td>01 February 2018</td></tr>
<tr><td></td><td></td><td>US   2018-0028654  A1</td><td>01 February 2018</td></tr>
<tr><td></td><td></td><td>US   2018-0028655  A1</td><td>01 February 2018</td></tr>
<tr><td></td><td></td><td>US   2018-0028656  A1</td><td>01 February 2018</td></tr>
<tr><td></td><td></td><td>US   2018-0028657  A1</td><td>01 February 2018</td></tr>
<tr><td></td><td></td><td>US   2018-0043018  A1</td><td>15 February 2018</td></tr>
<tr><td></td><td></td><td>US   2018-0043019  A1</td><td>15 February 2018</td></tr>
<tr><td></td><td></td><td>US   2018-0055929  A1</td><td>01 March 2018</td></tr>
<tr><td></td><td></td><td>US   2018-0055930  A1</td><td>01 March 2018</td></tr>
<tr><td></td><td></td><td>US   2018-0140701  A1</td><td>24 May 2018</td></tr>
<tr><td></td><td></td><td>US   2018-0311349  A1</td><td>01 November 2018</td></tr>
<tr><td></td><td></td><td>US   2018-0311350  A1</td><td>01 November 2018</td></tr>
<tr><td></td><td></td><td>US   2018-0311351  A1</td><td>01 November 2018</td></tr>
<tr><td></td><td></td><td>US   2018-0311352  A1</td><td>01 November 2018</td></tr>
<tr><td></td><td></td><td>US   2019-0060455  A1</td><td>28 February 2019</td></tr>
<tr><td></td><td></td><td>US   2019-0070292  A1</td><td>07 March 2019</td></tr>
<tr><td></td><td></td><td>US   2019-0070293  A1</td><td>07 March 2019</td></tr>
<tr><td></td><td></td><td>US   9340611  B2</td><td>17 May 2016</td></tr>
<tr><td></td><td></td><td>US   9340612  B2</td><td>17 May 2016</td></tr>
<tr><td></td><td></td><td>US   9346880  B2</td><td>24 May 2016</td></tr>
<tr><td></td><td></td><td>US   9382317  B2</td><td>05 July 2016</td></tr>
<tr><td></td><td></td><td>US   9682145  B2</td><td>20 June 2017</td></tr>
<tr><td></td><td></td><td>US   9707293  B2</td><td>18 July 2017</td></tr>
<tr><td></td><td></td><td>US   9724414  B2</td><td>08 August 2017</td></tr>
<tr><td></td><td></td><td>US   9724415  B2</td><td>08 August 2017</td></tr>
<tr><td></td><td></td><td>US   9731008  B2</td><td>15 August 2017</td></tr>
<tr><td></td><td></td><td>US   9731009  B2</td><td>15 August 2017</td></tr>
<tr><td></td><td></td><td>US   9737600  B2</td><td>22 August 2017</td></tr>
<tr><td></td><td></td><td>US   9757454  B2</td><td>12 September 2017</td></tr>
<tr><td></td><td></td><td>US   9770507  B2</td><td>26 September 2017</td></tr>
<tr><td></td><td></td><td>US   9782479  B2</td><td>10 October 2017</td></tr>
<tr><td></td><td></td><td>US   9782480  B2</td><td>10 October 2017</td></tr>
<tr><td></td><td></td><td>US   9789185  B2</td><td>17 October 2017</td></tr>
<tr><td></td><td></td><td>US   9808525  B2</td><td>07 November 2017</td></tr>
</table>

Form PCT/ISA/210 (patent family annex) (July 2019)

INTERNATIONAL SEARCH REPORT
Information on patent family members

International application No.

**PCT/KR2021/002983**

| Patent document cited in search report | | | Publication date (day/month/year) | Patent family member(s) | | | Publication date (day/month/year) |
|---|---|---|---|---|---|---|---|
| | | | | US | 9861695 | B2 | 09 January 2018 |
| | | | | UY | 35351 | A | 29 August 2014 |
| | | | | WO | 01-74143 | A1 | 11 October 2001 |
| | | | | WO | 2014-039903 | A2 | 13 March 2014 |
| | | | | WO | 2014-039903 | A3 | 26 June 2014 |
| KR | 10-2016-0068946 | A | 15 June 2016 | AU | 2014-339984 | A1 | 28 April 2016 |
| | | | | AU | 2014-339984 | B2 | 09 April 2020 |
| | | | | AU | 2020-204608 | A1 | 30 July 2020 |
| | | | | CA | 2926089 | A1 | 30 April 2015 |
| | | | | CN | 105611938 | A | 25 May 2016 |
| | | | | CN | 112107538 | A | 22 December 2020 |
| | | | | EP | 3060229 | A1 | 31 August 2016 |
| | | | | HK | 1221900 | A1 | 16 June 2017 |
| | | | | JP | 2016-535020 | A | 10 November 2016 |
| | | | | JP | 2019-116480 | A | 18 July 2019 |
| | | | | JP | 6483673 | B2 | 13 March 2019 |
| | | | | KR | 10-2020-0051844 | A | 13 May 2020 |
| | | | | KR | 10-2021-0041101 | A | 14 April 2021 |
| | | | | KR | 10-2238065 | B1 | 07 April 2021 |
| | | | | MX | 2016004605 | A | 14 November 2016 |
| | | | | RU | 2016119755 | A | 27 November 2017 |
| | | | | RU | 2016119755 | A3 | 28 April 2018 |
| | | | | SG | 10201803178 | A | 30 May 2018 |
| | | | | SG | 11201603206 | A | 30 May 2016 |
| | | | | US | 2015-0118249 | A1 | 30 April 2015 |
| | | | | US | 2019-0201535 | A1 | 04 July 2019 |
| | | | | US | 2020-0297855 | A1 | 24 September 2020 |
| | | | | WO | 2015-061584 | A1 | 30 April 2015 |
| EP | 3563867 | A1 | 06 November 2019 | AU | 2017-384942 | A1 | 08 August 2019 |
| | | | | BR | 112019013673 | A2 | 28 January 2020 |
| | | | | CL | 2019001818 | A1 | 29 November 2019 |
| | | | | CN | 110536698 | A | 03 December 2019 |
| | | | | CR | 20190316 | A | 04 September 2019 |
| | | | | EA | 201900360 | A1 | 30 December 2019 |
| | | | | EC | SP19048656 | A | 31 July 2019 |
| | | | | JP | 2020-506955 | A | 05 March 2020 |
| | | | | KR | 10-2019-0104043 | A | 05 September 2019 |
| | | | | MX | 2019007911 | A | 05 December 2019 |
| | | | | NI | 201900072 | A | 31 October 2019 |
| | | | | PE | 20191550 | A1 | 24 October 2019 |
| | | | | PH | 12019501530 | A1 | 24 February 2020 |
| | | | | RU | 0002665966 | C2 | 05 September 2018 |
| | | | | RU | 2016152691 | A | 02 July 2018 |
| | | | | RU | 2017146821 | A | 01 July 2019 |
| | | | | WO | 2018-124948 | A1 | 05 July 2018 |
| | | | | WO | 2018-124948 | A8 | 27 September 2018 |
| KR | 10-2017-0117166 | A | 20 October 2017 | AU | 2016-217806 | A1 | 05 October 2017 |
| | | | | BR | 112017016636 | A2 | 03 April 2018 |
| | | | | CA | 2976298 | A1 | 18 August 2016 |
| | | | | CN | 107635581 | A | 26 January 2018 |

Form PCT/ISA/210 (patent family annex) (July 2019)

**INTERNATIONAL SEARCH REPORT**
Information on patent family members

International application No.

**PCT/KR2021/002983**

| Patent document cited in search report | Publication date (day/month/year) | Patent family member(s) | | Publication date (day/month/year) |
|---|---|---|---|---|
| | | EP | 3256160 A1 | 20 December 2017 |
| | | JP | 2018-507202 A | 15 March 2018 |
| | | MX | 2017010400 A | 28 November 2017 |
| | | RU | 2017131618 A | 13 March 2019 |
| | | SG | 11201706505 A | 28 September 2017 |
| | | TW | 201632203 A | 16 September 2016 |
| | | US | 2018-0008707 A1 | 11 January 2018 |
| | | WO | 2016-128564 A1 | 18 August 2016 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6090382 A